(19) Europäisches Patentamt
European Patent Office
Office européen des brevets

(11) EP 3 992 617 A1

(12) **EUROPEAN PATENT APPLICATION**
published in accordance with Art. 153(4) EPC

(43) Date of publication:
**04.05.2022 Bulletin 2022/18**

(21) Application number: **20833397.1**

(22) Date of filing: **25.06.2020**

(51) International Patent Classification (IPC):
***G01N 21/64*** *(2006.01)* ***A61M 1/16*** *(2006.01)*

(52) Cooperative Patent Classification (CPC):
**A61M 1/16; G01N 21/64**

(86) International application number:
**PCT/JP2020/025023**

(87) International publication number:
**WO 2020/262534 (30.12.2020 Gazette 2020/53)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.06.2019 JP 2019118622**
**26.06.2019 JP 2019118624**

(71) Applicants:
- **Asahi Kasei Medical Co., Ltd.**
  **Tokyo 100-0006 (JP)**
- **The Kitasato Institute**
  **Tokyo 108-8641 (JP)**

(72) Inventors:
- **SHIMOIDE, Koji**
  **Tokyo 100-0006 (JP)**
- **HOSOYA, Noriyuki**
  **Tokyo 100-0006 (JP)**
- **KOBAYASHI, Hirosuke**
  **Sagamihara-shi, Kanagawa 252-0373 (JP)**
- **KOKUBO, Kenichi**
  **Sagamihara-shi, Kanagawa 252-0373 (JP)**
- **KOBAYASHI, Kozue**
  **Sagamihara-shi, Kanagawa 252-0373 (JP)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(54) **CONCENTRATION CALCULATION DEVICE AND BLOOD TREATMENT SYSTEM**

(57) A concentration calculation device of the present invention includes an emission unit that emits excitation light to dialysis drainage, a detection unit that detects fluorescence generated from the dialysis drainage, a classification unit that classifies a spectrum of the fluorescence detected by the detection unit into any of a plurality of predetermined groups, and a concentration calculation unit that calculates a concentration of albumin contained in the dialysis drainage on the basis of a calibration model that corresponds to a group of the fluorescence spectrum classified by the classification unit among a plurality of calibration models provided in advance corresponding to each of the plurality of groups, and fluorescence intensities of a plurality of wavelengths in a predetermined wavelength range of a fluorescence spectrum detected by the detection unit.

Fig. 1

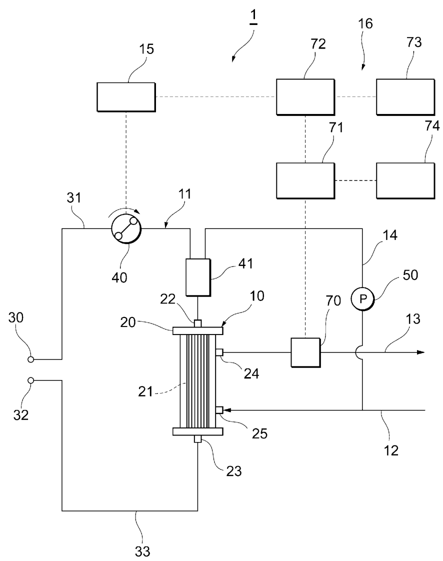


EP 3 992 617 A1

## Description

### Technical Field

This application is based on Japanese Patent Application No. 2019-118622, filed June 26, 2019, and Japanese Patent Application No. 2019-118624, filed June 26, 2019, the content of which is incorporated herein by reference.
The present invention relates to a concentration calculation device and a blood treatment system.

### Background Art

Hemodialysis, which is a method of treating patients with renal failure, and hemodiafiltration are currently widely used treatment methods. In these treatment methods, from blood that has flowed into a dialyzer (filter), waste products are discharged into a fresh dialysate that has also flowed into a dialyzer by diffusion and filtration through a dialysis membrane.
Many patients with renal failure often complain of various symptoms such as pruritus, irritation, and osteoarthritis. In order to relieve such symptoms, $\alpha$1-microglobulin (MG) is actively removed as an indicator substance. However, according to active removal of $\alpha$1-MG, albumin required for a living body inevitably leaks into the dialysis drainage because the size thereof is close to $\alpha$1-MG. In performing dialysis treatment three times a week, an albumin management method including measuring a serum albumin concentration of a patient once or twice a month and determining an albumin level is currently generally used.
In view of the above circumstances, a means of determining a concentration of albumin in dialysis drainage in real time is required in the field of dialysis treatment.
As a method of measuring a concentration of a target substance such as albumin in dialysis drainage, Patent Documents 1 and 2 disclose a method of emitting UV rays and measuring a concentration of a substance from a fluorescence intensity.
As a method of calculating a concentration of a target substance in dialysis drainage, Patent Document 3 discloses a method of calculating a concentration of a target substance, with the method including emitting UV rays and excluding the influence of contaminants (indoxyl sulfate, etc.) from a fluorescence intensity.
In addition, as a method of calculating a concentration of a target substance, Patent Document 4 discloses a method in which linearly polarized light is emitted, an intensity of the emitted fluorescence on a first polarizing surface is detected, an intensity on a second polarizing surface different from the first polarizing surface is detected, the anisotropy of these fluorescences is specified, and based on both the anisotropy and the intensity, the concentration of one of these is calculated.
In addition, as a method of calculating a concentration of albumin, Patent Document 5 discloses a method in which an absorbance of dialysis drainage is measured, separation is then performed with a filter, which is an albumin separation unit, an absorbance is additionally measured, and a concentration of albumin is calculated from the change in absorbance before and after passing through the filter.

Citation List

Patent Document

Patent Document 1: European Patent Specification No. 2579910

Patent Document 2: European Patent Specification No. 2579911

Patent Document 3: Japanese Translation of PCT Application No. 2014-518517

Patent Document 4: Japanese Translation of PCT Application No. 2015-521492

Patent Document 5: Patent Publication JP-A-2015-146837

**Summary**

Technical Problem

However, in the methods disclosed in Patent Documents 1 and 2, in the dialysis drainage containing various contaminants, it is difficult to establish an additivity in the fluorescence intensity between fluorescent substances, and there are provided a fluorescence resonance energy transfer phenomenon and a substance that adsorbs fluorescence of a target substance, and thus it cannot be said that a correlation between the fluorescence intensity and the concentration of the target substance is high, and it is not possible to measure a substance concentration with high accuracy at an excitation wavelength of 280 nm in Patent Documents 1 and 2.

In the method disclosed in Patent Document 3, in the case of albumin, the fluorescence of albumin is due to the tryptophan residue, and there is also provided tryptophan as an amino acid alone in the dialysis drainage. The fluorescences of albumin and tryptophan at an excitation wavelength of 280 nm and 295 nm are very similar, and in the method of Patent Document 3, linear superposition is not established and separation is not possible. Therefore, it is not possible to calculate an albumin concentration with high accuracy.

In the fluorescence polarization method disclosed in Patent Document 4, since the anisotropy differs depending on the molecular weight of the fluorescent substance, the device tends to become complicated and expensive due to provision of a rotating part in order to detect fluorescence thereof. In addition, because fluorescent substance proteins contain one or more of tryptophan residues, tyrosine residues, and phenylalanine residues in many cases, low molecular weight proteins contained in the dialysis drainage have similar molecular weights, and they have similar excitation wavelengths and fluorescence wavelengths, classification thereof based on the anisotropy alone is difficult.

In addition, in the method disclosed in Patent Document 5, a filter has to be used for an albumin separation unit, and in addition to problems of a filter replacement frequency and a filter price, it cannot be denied that there are many inconveniences in terms of practical use due to the need for more accurate control of a filter flow rate and a filtration flow rate, and the complexity of the device.

The present invention has been made in view of the above circumstances and an object of the present invention is to provide a concentration calculation device that can calculate a concentration of a substance, such as albumin, with high accuracy without complicating the device, and a blood treatment system.

Solution to Problem

The inventors conducted extensive studies, and as a result, found that the above problems can be addressed by classifying a predetermined element that affects a calibration model for concentration calculation into one of a plurality of predetermined groups, and calculating a concentration of a substance using a calibration model corresponding to the classified predetermined element group, and completed the present invention.

That is, the present invention includes the following aspects.

(1) A concentration calculation device, including: an emission unit that emits excitation light to a concentration calculation target; a detection unit that detects fluorescence generated from the concentration calculation target; a classification unit that classifies a predetermined element that affects concentration calculation into any of a plurality of predetermined groups; and a concentration calculation unit that calculates a concentration of a substance contained in the concentration calculation target on the basis of a calibration model that corresponds to a group of the predetermined element classified by the classification unit among a plurality of calibration models provided in advance corresponding to each of the plurality of groups, and fluorescence intensities of a plurality of wavelengths in a predetermined wavelength range of a fluorescence spectrum detected by the detection unit.

(2) The concentration calculation device according to (1), wherein the element is a spectrum of the fluorescence detected by the detection unit.

(3) The concentration calculation device according to (2), wherein the plurality of groups are divided, based on a feature amount obtained by performing principal-component analysis on the fluorescence spectrum.

(4) The concentration calculation device according to any one of (1) to (3), wherein the concentration calculation target is dialysis drainage discharged in dialysis treatment.

(5) The concentration calculation device according to (1), wherein the concentration calculation target is dialysis drainage discharged in dialysis treatment, and wherein the element is any of a spectrum of the fluorescence, a time elapsed from when the dialysis treatment starts, a patient to be treated with dialysis, a patient treatment method, a type of a filter of a dialyzer used in the dialysis treatment, an intensity of fluorescence detected by the detection unit, a maximum value in a predetermined wavelength range of the fluorescence spectrum, and one or more setting parameters for detection of the detection unit.

(6) The concentration calculation device according to (4) or (5), wherein the substance is albumin in the dialysis drainage.

(7) The concentration calculation device according to any one of (4) to (6), wherein parameter values related to concentration calculation are set so that the relationship between a %error A of a substance concentration allowed in any time block of a first half of the dialysis treatment and a %error B of the measured concentration allowed in any time block of a second half of the dialysis treatment is $A \leq B$.

(8) The concentration calculation device according to any one of (4) to (7), wherein an approximate curve of an estimated substance concentration represented by Formula (1) is obtained using at least 3 or more points of the real time $t_n$ of the concentration of the substance in the dialysis drainage up to the calculation point of the substance concentration at an immediately previous time $t_{n-1}$, and when an estimated difference rate $EF(t_n)$ represented by Formula (4) obtained by dividing an estimated difference $E(t_n)$ defined by Formula (2) from an estimated substance concentration $C(t_n)$ in Formula (1) by the root mean square $RMSD(t_{n-1})$ of an estimated difference $E(t_{n-1})$ to the real time $t_{n-1}$ represented by Formula (3) satisfies $-1.0 \leq EF(t_n) \leq 1.0$ at t<a dialysis time of 60 minutes, satisfies $-1.2 \leq EF(t_n) \leq 1.2$ at a dialysis time of 60 minutes≤t<a dialysis time of 120 minutes, and satisfies $-1.5 \leq EF(t_n) \leq 1.5$ at a dialysis time of 120 minutes≤t, the substance concentration calculated at the real time $t_n$ is used:

$$C(t)=A\times t^{\wedge}(-B)\cdots(1)$$

$$E(t_n)=(\text{calculated concentration at real time } t_n)-C(t_n)\cdots(2)$$

$$RMSD(t_{n-1})=\{[\Sigma(E(t_i)^{\wedge}2)]/(n-1)\}^{\wedge}0.5\cdots(3)$$

$$EF(t_n)=E(t_n)/RMSD(t_{n-1})\cdots(4)$$

C: estimated concentration of the substance in the dialysis drainage [mg/dL], A: constant, t: time [min], B: constant, E: estimated difference, subscript i: i-th calculated concentration, subscript n: n-th calculated concentration.

(9) The concentration calculation device according to any one of (1) to (8), further including a calibration model creation unit that creates a plurality of calibration models by performing multivariate analysis on the fluorescence spectrum for each of the plurality of groups.

(10) The concentration calculation device according to (9), wherein the multivariate analysis is any of partial least squares (PLS) regression analysis, principal component regression analysis, multiple regression analysis, support vector machine regression analysis, and machine learning analysis.

(11) The concentration calculation device according to any one of (1) to (10), further including a storage unit that stores a plurality of substance concentrations calculated by the concentration calculation unit at a plurality of time points.

(12) The concentration calculation device according to (11), further including an integration value calculation unit that calculates a time integration value of a product of each substance concentration corresponding to a plurality of time points stored in the storage unit and a flow rate of the concentration calculation target.

(13) The concentration calculation device according to (11) or (12), further including a change rate calculation unit that calculates a rate of change (dC/dt, C: substance concentration, t: time), with respect to time, in substance concentrations corresponding to a plurality of time points stored in the storage unit.

(14) The concentration calculation device according to any one of (11) to (13), further including a difference calculation unit that calculates a difference between each of the substance concentrations corresponding to a plurality of time points stored in the storage unit and a predetermined value.

(15) The concentration calculation device according to any one of (11) to (14), further including an estimated value calculation unit that calculates a formula of the substance concentration over time, based on each of the substance concentrations corresponding to a plurality of times stored in the storage unit and calculates an estimated value of a total amount of the substance contained in the concentration calculation target, based on the formula over time.

(16) The concentration calculation device according to any one of (1) to (15), further including a display unit that displays information on the substance concentration calculated by the concentration calculation unit.

(17) The concentration calculation device according to any one of (1) to (16), wherein the concentration calculation target to which the excitation light is emitted is a continuous flow having a flow rate of 10 mL/min or more and 1,000 mL/min or less.

(18) A blood treatment system including the concentration calculation device according to any one of (1) to (17).

(19) A concentration calculation device, including: an emission unit that emits excitation light to an emission target containing albumin; a detection unit that detects fluorescence generated from the emission target; and a concentration calculation unit that calculates a concentration of albumin contained in the emission target, based on fluorescence intensities of a plurality of wavelengths in a predetermined wavelength range of a fluorescence spectrum detected by the detection unit and a calibration model provided in advance, wherein the excitation light includes light having a wavelength of 300 nm or more and 400 nm or less, which excites fluorescence exhibiting a sub-peak of the albumin.

(20) The concentration calculation device according to (19), wherein the excitation light includes light having a wavelength of 310 nm or more and 380 nm or less.

(21) The concentration calculation device according to (19) or (20), wherein the predetermined wavelength range of the fluorescence spectrum includes a range from a wavelength obtained by adding 10 nm to a wavelength lower limit of the excitation light to a wavelength obtained by adding 450 nm to a wavelength upper limit of the excitation light.

(22) The concentration calculation device according to (19) or (20), wherein the predetermined wavelength range of the fluorescence spectrum includes a range from a wavelength obtained by adding 10 nm to a wavelength lower limit of the excitation light to a wavelength obtained by adding 400 nm to a wavelength upper limit of the excitation light.

(23) The concentration calculation device according to any one of (19) to (22), further including a calibration model creation unit that creates the calibration model by performing multivariate analysis.

(24) The concentration calculation device according to (23), wherein the multivariate analysis is any of partial least squares (PLS) regression analysis, principal component regression analysis, multiple regression analysis, support vector machine regression analysis, and machine learning analysis.

(25) The concentration calculation device according to any one of (19) to (24), wherein the albumin concentration is calculated by formula below:

$$C=a_1\times I_1+a_2\times I_2+\cdots+a_n\times I_n+K$$

C: calculated albumin concentration, $a_n$: coefficient, $I_n$: fluorescence intensity, K: constant, subscript n: a natural number in which each wavelength from which the fluorescence spectrum is acquired is numbered from the shortest.

(26) The concentration calculation device according to any one of (19) to (25), wherein the emission target is dialysis drainage discharged in dialysis treatment.

(27) The concentration calculation device according to (26), further including a storage unit that stores a plurality of albumin concentrations calculated by the concentration calculation unit in the dialysis treatment.

(28) The concentration calculation device according to (27), further including an integration value calculation unit that calculates an integration value of a product of the albumin concentration stored in the storage unit and a dialysis drainage flow rate after the dialysis treatment starts.

(29) The concentration calculation device according to (27) or (28), further including a change rate calculation unit that calculates a rate of change (dC/dt, C: albumin concentration, t: time), with respect to time, in the albumin concentration stored in the storage unit.

(30) The concentration calculation device according to any one of (27) to (29), further including a difference calculation unit that calculates a difference between the albumin concentration stored in the storage unit and a predetermined value.

(31) The concentration calculation device according to any one of (27) to (30), further including an estimated value calculation unit that calculates a formula of the albumin concentration over time after the dialysis treatment starts, based on the albumin concentration stored in the storage unit, and calculates an estimated value of a total amount of albumin in the dialysis drainage from when the dialysis treatment starts until the dialysis treatment ends, based on the formula over time.

(32) The concentration calculation device according to any one of (26) to (31), further including a display unit that displays information on the albumin concentration calculated by the concentration calculation unit.

(33) The concentration calculation device according to any one of (26) to (32), wherein parameter values related to concentration calculation are set so that a relationship between a %error A of an albumin concentration allowed in any time block of a first half of the dialysis treatment and a %error B of the measured concentration allowed in any time block of a second half of the dialysis treatment is A≤B.

(34) The concentration calculation device according to any one of (26) to (33), wherein an approximate curve of the estimated albumin concentration represented by Formula (1) is obtained using at least 3 or more points of the real time $t_n$ of the concentration of albumin in the dialysis drainage up to a calculation point of the albumin concentration at an immediately previous time $t_{n-1}$, and when an estimated difference rate $EF(t_n)$ represented by Formula (4) obtained by dividing an estimated difference $E(t_n)$ defined by Formula (2) from an estimated albumin concentration $C(t_n)$ in Formula (1) by the root mean square $RMSD(t_{n-1})$ of an estimated difference $E(t_{n-1})$ to the real time $t_{n-1}$ represented by Formula (3) satisfies $-1.0 \le EF(t_n) \le 1.0$ at t<a dialysis time of 60 minutes, satisfies $-1.2 \le EF(t_n) \le 1.2$ at a dialysis time of 60 minutes≤t<a dialysis time of 120 minutes, and satisfies $-1.5 \le EF(t_n) \le 1.5$ at a dialysis time of 120 minutes≤t, the albumin concentration calculated at the real time $t_n$ is used:

$$C(t)=A\times t^{\wedge}(-B)\cdots(1)$$

$$E(t_n)=(\text{calculated concentration at real time } t_n)-C(t_n)\cdots(2)$$

$$RMSD(t_{n-1})=\{[\Sigma(E(t_i)^{\wedge}2)]/(n-1)\}^{\wedge}0.5\cdots(3)$$

$$EF(t_n)=E(t_n)/RMSD(t_{n-1})\cdots(4)$$

C: estimated concentration of albumin in the dialysis drainage [mg/dL], A: constant, t: time [min], B: constant, E: estimated difference, subscript i: i-th calculated concentration, subscript n: n-th calculated concentration.

(35) The concentration calculation device according to any one of (26) to (34), wherein the dialysis drainage to which the excitation light is emitted is a continuous flow having a flow rate of 10 mL/min or more and 1,000 mL/min or less.

(36) A blood treatment system, including the concentration calculation device according to any one of (19) to (35).

## Advantageous Effects of Invention

According to the present invention, it is possible to provide a concentration calculation device that can calculate a substance concentration with high accuracy without complicating the device, and a blood treatment system.

**Brief Description of Drawings**

Fig. 1 is an explanatory diagram showing an example of a configuration of a dialysis system according to a first embodiment.

Fig. 2 is an explanatory diagram showing an example of a configuration of an optical monitor.

Fig. 3 is a diagram showing an excitation wavelength at which a fluorescence characteristic of albumin exhibits a sub-peak.

Fig. 4 is a graph for illustrating classification of a plurality of groups.

Fig. 5 is a block diagram of a concentration calculation device.

Fig. 6 is a block diagram of a numerical value computation unit.

Fig. 7 is a graph for illustrating an integration value of an albumin leakage amount.

Fig. 8 is a graph for illustrating a rate of change (dC/dt) in an albumin concentration with respect to time.

Fig. 9 is a graph for illustrating the difference between an albumin concentration and a predetermined value.

Fig. 10 is a graph for illustrating an estimation curve of an albumin leakage amount.

Fig. 11 is a diagram showing wavelengths in fluorescence spectrums.

Fig. 12 is a graph for illustrating an error in the calculated albumin concentration.

Fig. 13 is a graph showing a change in the albumin concentration in general dialysis drainage.

Fig. 14 is a diagram showing fluorescence spectrums.

Fig. 15 is a graph showing the calculated concentration and the measured concentration of albumin.

Fig. 16 is a graph showing the results obtained by performing principal-component analysis on fluorescence spectrums.

Fig. 17 is an explanatory diagram showing an example of a configuration of a dialysis system according to a second embodiment.

Fig. 18 is an explanatory diagram showing an example of a configuration of an optical monitor.

Fig. 19 is a diagram showing an excitation wavelength at which a fluorescence characteristic of albumin exhibits a sub-peak.

Fig. 20 is a block diagram of the concentration calculation device.

Fig. 21 is a block diagram of a numerical value computation unit.

Fig. 22 is a graph for illustrating an integration value of an albumin leakage amount.

Fig. 23 is a graph for illustrating a rate of change (dC/dt) in the albumin concentration with respect to time.

Fig. 24 is a graph for illustrating the difference between an albumin concentration and a predetermined value.

Fig. 25 is a graph for illustrating an estimation curve of an albumin leakage amount.

Fig. 26 is a diagram showing wavelengths in fluorescence spectrums.

Fig. 27 is a table showing a fluorescence wavelength range, a correlation coefficient, and a root mean square error.

Fig. 28 is a graph for illustrating an error of the calculated albumin concentration.

Fig. 29 is a graph showing the change in an albumin concentration of general dialysis drainage.

Fig. 30 is a diagram showing fluorescence spectrums.

Fig. 31 is a graph showing an actual concentration and an estimated concentration of albumin in Example 1.

Fig. 32 is a graph showing an actual concentration and an estimated concentration of albumin in comparative examples.

Fig. 33 is a table showing an excitation wavelength and a root mean square error.

Fig. 34 is a graph showing the change in the albumin concentration over time.

**Description of Embodiments**

Hereinafter, examples of preferable embodiments of the present invention will be described with reference to the drawings. Here, positional relationships such as above, below, left and right in the drawing are based on the positional relationship shown in the drawings unless otherwise specified. Dimensional ratios in the drawings are not limited to the illustrated ratios. In addition, the following embodiments are examples for explaining the present invention, and there is no intention to limit the present invention to only the embodiments. In addition, the present invention can be variously modified without departing from the scope and spirit thereof.

First embodiment

<Dialysis system>

Fig. 1 is an explanatory diagram showing a configuration outline of a dialysis system 1 as a blood treatment system on which a concentration calculation device is mounted according to the present embodiment.

The dialysis system 1 includes, for example a dialyzer 10, a blood circuit 11, a dialysate circuit 12, a drainage circuit 13, a fluid substitution circuit 14, a control device 15 and a concentration calculation device 16.

The dialyzer 10 is, for example, a hollow fiber module having a built-in hollow fiber membrane, and can separate unnecessary constituents from blood. The dialyzer 10 has a cylindrical container 20, and a plurality of hollow fiber membranes 21 are disposed inside the cylindrical container 20 in a longitudinal direction. The hollow fiber membrane 21 can separate unnecessary constituents from blood. An inlet 22 and an outlet 23 that lead to the space inside the tube (blood side) of the hollow fiber membrane 21 are provided above and below the cylindrical container 20. Two entrances 24 and 25 that lead to the space outside the tube (dialysate side) of the hollow fiber membrane 21 are provided at the side part of the cylindrical container 20.

**The** blood circuit 11 includes, for example, a blood removal line 31 that connects a blood removal unit 30 and the dialyzer 10, and a blood return line 33 that connects the dialyzer 10 and a blood return unit 32. The blood removal line 31 and the blood return line 33 are mainly composed of a soft tube. The blood removal line 31 is connected to the inlet 22 of the dialyzer 10, and the blood return line 33 is connected to the outlet 23 of the dialyzer 10.

For example, a blood pump 40 is provided at the blood removal line 31. In addition, a drip chamber 41 is connected to the blood removal line 31. The drip chamber 41 may not be provided.

The dialysate circuit 12 is connected to the entrance 25 of the dialyzer 10 from a dialysate supply source (not shown). The drainage circuit 13 is connected to a drainage unit (not shown) from the entrance 24 of the dialyzer 10. A supply/drainage pump (not shown) that supplies a dialysate to the dialyzer 10 through the dialysate circuit 12 and drains a dialysate from the dialyzer 10 through the drainage circuit 13 is provided at the dialysate circuit 12 and the drainage circuit 13.

For example, the fluid substitution circuit 14 is connected to the drip chamber 41 (the blood circuit 11) from the dialysate circuit 12. When the drip chamber 41 is not provided, the fluid substitution circuit 14 is directly connected to the blood removal line 31. A fluid substitution pump 50 is provided at the fluid substitution circuit 14.

The control device 15 is, for example, a computer. For example, when a CPU executes a program stored in a storage unit, operations of the blood pump 40 and the fluid substitution pump 50 are controlled, and dialysis processing for dialysis treatment can be performed. Here, communication between the control device 15 and various devices (the blood pump 40 and the fluid substitution pump 50) may be performed via a wire such as a communication cable or may be performed in a wireless manner such as through Bluetooth (registered trademark).

In the dialysis treatment, in the blood circuit 11, the patient's blood is sent from the blood removal unit 30 to the space inside the tube of the hollow fiber membrane 21 of the dialyzer 10, passes through the dialyzer 10, and is then returned to the patient from the blood return unit 32. In this case, the dialysate passes through the dialysate circuit 12, is sent to the space outside the tube of the hollow fiber membrane 21 of the dialyzer 10, and then drained through the drainage circuit 13. In the dialyzer 10, mainly unnecessary constituents in the blood flowing through the space inside the tube of the hollow fiber membrane 21 flow out to the space outside the tube (dialysate side) through the hollow fiber membrane 21, and are discharged together with the dialysate. A substitution fluid (dialysate) of the dialysate circuit 12 is supplied to the blood circuit 11 through the fluid substitution circuit 14 and a predetermined constituent is refilled in the blood. Here, hemodiafiltration in which a substitution fluid is refilled and hemodialysis in which a substitution fluid is not refilled can be performed.

<Concentration calculation device>

The concentration calculation device 16 includes an optical monitor 70 that emits excitation light to dialysis drainage that is a concentration calculation target and detects fluorescence generated from the dialysis drainage, a classification unit 71 that classifies the fluorescence spectrum acquired by the optical monitor 70 into one of a plurality of predetermined groups as a predetermined element that affects concentration calculation, a concentration calculation unit 72 that calculates a concentration of albumin as a substance contained in the dialysis drainage based on a calibration model corresponding to the group of the fluorescence spectrum classified by the classification unit 71 and fluorescence intensities of a plurality of wavelengths in a predetermined wavelength range of the spectrum of fluorescence detected by the optical monitor 70, a display unit 73, and an input unit 74. Here, communication between the optical monitor 70, the classification unit 71, the concentration calculation unit 72, the display unit 73 and the input unit 74 may be performed via a wire such as a communication cable, or may be performed in a wireless manner such as through Bluetooth (registered trademark).

For example, as shown in Fig. 2, the optical monitor 70 includes an emission unit 80 that emits excitation light to the dialysis drainage in the pipe of the drainage circuit 13 and a detection unit 81 that detects fluorescence generated from the dialysis drainage.

The emission unit 80 can emit light having a wavelength of 300 nm or more and 400 nm or less, which excites fluorescence exhibiting a sub-peak of albumin shown in Fig. 3. A light source of the emission unit 80 is not particularly limited, and for example, a halogen lamp, a xenon lamp, a deuterium lamp, or an LED may be used. The wavelength of excitation light emitted from the emission unit 80 is preferably 310 nm or more and 380 nm or less, and more preferably 330 nm or more and 350 nm or less.

The detection unit 81 detects fluorescence generated from the dialysis drainage to obtain a fluorescence spectrum. The detection unit 81 may detect fluorescence in a wavelength range of 310 nm or more and 850 nm or less. The fluorescence wavelength detected by the detection unit 81 is preferably 310 nm or more and 700 nm or less and more preferably 320 nm or more and 650 nm or less. In addition, for example, the emission unit 80 and the detection unit 81 are disposed in directions perpendicular to each other with respect to the pipe of the drainage circuit 13, and acquire fluorescence at an angle perpendicular to the excitation light. Here, the disposition of the emission unit 80 and the detection unit 81 and the shape of the drainage circuit 13 are not limited thereto.

The classification unit 71 is, for example, a computer, and classifies the spectrum of fluorescence detected by the detection unit 81 into one of a plurality of predetermined groups. For example, as shown in Fig. 4, the plurality of groups are divided based on the feature amount obtained by performing principal-component analysis on the fluorescence spectrum in advance, and the plurality of groups are divided into four groups A, B, C, and D according to a value of a first component (i component) of a component factor showing the most characteristic feature of the fluorescence spectrum obtained by principal-component analysis and a value of a second component (k component), which is a component factor showing the next characteristic feature of the fluorescence spectrum. Specifically, in a graph in which the horizontal axis represents the first component and the vertical axis represents the second component, the first component and the second component are positive in the group A, the first component is negative and the second component is positive in the group B, the first component is positive and the second component is negative in the group C, and the first component and the second component are negative in the group D. In addition, the classification unit 71 may not be incorporated into the main body of the concentration calculation device 16. It is also possible to communicate fluorescence spectrum data detected by the detection unit 81, classify it on the cloud, and communicate with the concentration calculation unit 72.

Fig. 5 shows a block diagram of the concentration calculation unit 72. The concentration calculation unit 72 is, for example, a computer. The concentration calculation unit 72 includes, for example, a concentration computation unit 90 that calculates a concentration of albumin in the dialysis drainage using a calibration model corresponding to a group of a fluorescence spectrum classified by the classification unit 71 among a plurality of calibration models provided in advance corresponding to the plurality of groups A to D, and fluorescence intensities of a plurality of wavelengths in a predetermined wavelength range of the fluorescence spectrum acquired by the optical monitor 70, a storage unit 91 that stores the albumin concentration obtained by the concentration computation unit 90, a numerical value computation unit 92 that computes various numerical values to be described below using the albumin concentration stored in the storage unit 91, and a calibration model creation unit 93 that creates a plurality of calibration models for each of the groups A to D.

For example, the calibration model creation unit 93 acquires fluorescence spectrum information of the groups A to D from the optical monitor 70 in advance, multivariately analyzes the information, and creates calibration models Ma, Mb, Mc, and Md corresponding to the groups A to D. The multivariate analysis for creating the calibration models Ma to Md may be any of partial least squares (PLS) regression analysis, principal component regression analysis, multiple regression analysis, support vector machine regression analysis, and machine learning analysis. An example of the calibration models Ma to Md is shown below. The calibration models Ma to Md can be expressed as a formula for obtaining a calculated concentration C by multiplying a fluorescence intensity of each wavelength of the obtained fluorescence spectrum by a coefficient and calculating their sum. The calibration models Ma to Md are specifically expressed as in Formula (5).

$$C=a_1\times I_1+a_2\times I_2+\cdots+a_n\times I_n+K\cdots(5)$$

C: calculated albumin concentration, $a_n$: coefficient, $I_n$: fluorescence intensity, K: constant, and subscript n: a natural number in which each wavelength from which the fluorescence spectrum is acquired is numbered from the shortest (example: if the wavelengths are 300 nm, 310 nm, 320 nm, 400 nm, the subscripts are 1, 2, 3, ···, 11)

The calibration models Ma to Md created by the calibration model creation unit 93 are stored in, for example, the storage unit 91, and are used as parameters in the computation process in the concentration computation unit 90 and the numerical value computation unit 92. Here, the calibration model obtained in advance may be stored in the storage unit 91 and this may be used.

The concentration computation unit 90 acquires a fluorescence spectrum of the dialysis drainage from the optical monitor 70 during dialysis treatment in real time, and computes an albumin concentration using fluorescence intensities of a plurality of wavelengths in a predetermined wavelength range of the fluorescence spectrum and the calibration models Ma to Md corresponding to the groups A to D classified by the classification unit 71.

The numerical value computation unit 92 computes various numerical values during dialysis treatment in real time by incorporating a value of the albumin concentration stored in the storage unit 91. As shown in Fig. 6, for example, the numerical value computation unit 92 includes an integration value calculation unit 100 that calculates an integration value of a product of an albumin concentration and a dialysis drainage flow rate after the dialysis treatment starts, a change rate calculation unit 101 that calculates a rate of change in the albumin concentration, a difference calculation unit 102 that calculates a difference between an albumin concentration and a predetermined value, and an estimated value calculation unit 103 that calculates an estimated value of a total amount of albumin in the dialysis drainage from when the dialysis treatment starts until the dialysis treatment ends.

As shown in Fig. 7, the integration value calculation unit 100 calculates an albumin leakage amount at that time from the product of the value of the albumin concentration and the dialysis drainage flow rate after the dialysis treatment starts, and calculates an integration value of the albumin leakage amount from a curve of the albumin leakage amount over time. This integration value is an albumin leakage amount (the shaded part in Fig. 7) up to that time from when the dialysis treatment starts.

As shown in Fig. 8, in the change rate calculation unit 101, the slope of a time change curve of the albumin concentration is a rate of change (dC/dt) in albumin over time.

As shown in Fig. 9, a separately predetermined value in the difference calculation unit 102 is, for example, an albumin concentration calculated from a preset allowable albumin leakage amount, and if the albumin concentration exceeds this, it becomes a threshold value indicating excessive albumin leakage. In this case, the difference calculation unit 102 calculates a difference between an albumin concentration and a threshold value thereof.

First, the estimated value calculation unit 103 obtains A and B shown in Formula (1) from at least 3 or more points of the albumin concentration. According to Formula (1), an albumin leakage amount estimation curve shown in Fig. 10 can be obtained. Then, based on Formula (1), an albumin leakage amount after the dialysis treatment starts is estimated, and an estimated value of a total amount of albumin (total leakage amount) per treatment during the dialysis treatment is calculated.

$$C(t)=A\times t^{\wedge}(-B)\cdots(1)$$

C: concentration in dialysis drainage [mg/dL], A: constant, t: time, [min], B: constant

The display unit 73 shown in Fig. 5 is, for example, a panel display that displays the albumin concentration calculated by the concentration computation unit 90 and various numerical values obtained by the numerical value computation unit 92, and various warnings. The warnings are issued, for example, when the albumin concentration calculated by the concentration computation unit 90 exceeds a predetermined range, when the integration value of the albumin leakage amount calculated by the integration value calculation unit 100 exceeds a predetermined range, when the rate of change in the albumin concentration calculated by the change rate calculation unit 101 exceeds a predetermined range, when the difference between the albumin concentration calculated by the difference calculation unit 102 and a threshold value thereof exceeds a predetermined range, and when the estimated value of the total amount of albumin per treatment calculated by the estimated value calculation unit 103 exceeds a predetermined range.

**The** input unit 74 has a function of inputting information required for classification by the classification unit 71 and information required for computing the albumin concentration by the concentration calculation unit 72, creating calibration models, and computing various numerical values from the outside. Here, the control device 15, the classification unit 71, the concentration calculation unit 72, the display unit 73 and the input unit 74 described above may be units and functions realized by the same computer.

Next, operations of the concentration calculation device 16 configured as described above will be described.

The concentration calculation device 16 continuously or intermittently calculates an albumin concentration in the dialysis drainage of the drainage circuit 13 during the dialysis treatment in real time. The dialysis drainage is a continuous flow with a flow rate of 10 mL/min or more and 1,000 mL/min or less.

Specifically, the emission unit 80 of the optical monitor 70 emits excitation light to the drainage circuit 13, and the detection unit 81 detects fluorescence generated from the dialysis drainage. The excitation light emitted from the emission unit 80 includes light having a wavelength of 300 nm or more and 400 nm or less, which excites fluorescence exhibiting a sub-peak of albumin.

As shown in Fig. 11, the spectrum of fluorescence detected by the detection unit 81 is in, for example, a wavelength range of 310 nm or more and 850 nm or less. In the detection unit 81, parameters related to measurement are adjusted based on the numerical value of the intensity maximum value in a wavelength range of 380 nm or more and 480 nm or less of the obtained fluorescence spectrum, and measurement is performed within the measurement upper limit of the sensor of the detection unit 81.

Next, the classification unit 71 classifies the spectrum of fluorescence detected by the detection unit 81 into a plurality of predetermined groups A to D. Specifically, the spectrums of fluorescence detected by the detection unit 81 are subjected to principal-component analysis to obtain values of a first component (i component) and a second component (k component), and the spectrums of fluorescence detected by the detection unit 81 are classified into a group corresponding to the values of the first component (i component) and the second component (k component) in the groups A to D.

The concentration computation unit 90 of the concentration calculation unit 72 calculates a concentration of albumin contained in the dialysis drainage based on the calibration model corresponding to the group of the fluorescence spectrum classified by the classification unit 71 among four calibration models Ma to Md provided in advance corresponding to the four groups A to D, and fluorescence intensities of a plurality of wavelengths in a predetermined wavelength range of the spectrum of fluorescence detected by the detection unit 81. For example, when the spectrum of fluorescence detected by the detection unit 81 is classified into the group A, the concentration computation unit 90 calculates an albumin concentration using the calibration model Ma stored in the storage unit 91. The concentration computation unit 90 continuously or intermittently calculates an albumin concentration in the dialysis drainage during the dialysis treatment in real time. The albumin concentration calculated by the concentration computation unit 90 is stored in the storage unit 91 each time. For example, the albumin concentration is displayed on the display unit 73 in real time.

Here, the calibration models Ma to Md are created in advance before the dialysis treatment starts in the calibration model creation unit 93 and stored in the storage unit 91. For example, the calibration model creation unit 93 acquires fluorescence spectrums of the dialysis drainage with different albumin concentrations from the optical monitor 70, and creates calibration models Ma to Md by multivariate analysis.

The numerical value computation unit 92 takes a plurality of albumin concentrations at respective time points calculated by the concentration computation unit 90 from the storage unit 91 and computes various numerical values. The integration value calculation unit 100 computes a time integration value of the product of the albumin concentration and the dialysis drainage flow rate after the dialysis treatment starts, the change rate calculation unit 101 computes a rate of change in the albumin concentration, the difference calculation unit 102 computes a difference between the albumin concentration and a predetermined value, and the estimated value calculation unit 103 computes an estimated value of the total amount of albumin in the dialysis drainage from when the dialysis treatment starts until the dialysis treatment ends. For example, these values are displayed on the display unit 73. In addition, if the albumin concentration, the integration value of the albumin leakage amount, the rate of change in the albumin concentration, the difference between the albumin concentration and the threshold value, or the estimated amount of the albumin leakage amount per treatment is not within an appropriate range, a warning is displayed on the display unit 73.

According to the present embodiment, the classification unit 71 classifies the spectrum of fluorescence detected by the detection unit 81 into one of a plurality of predetermined groups A to D, and the concentration calculation unit 72 calculates a concentration of albumin contained in the dialysis drainage based on the calibration model corresponding to the group of the fluorescence spectrum classified by the classification unit 71 among a plurality of calibration models Ma to Md provided in advance corresponding to the plurality of groups A to D and fluorescence intensities of a plurality of wavelengths in a predetermined wavelength range of the spectrum of fluorescence detected by the detection unit 81. As a result, it is possible to calculate the albumin concentration with high accuracy without complicating the device.

Since the plurality of groups A to D are divided based on the feature amount obtained by performing principal-component analysis on the fluorescence spectrum, it is possible to calculate the albumin concentration with high accuracy. Here, in the present embodiment, regarding the feature amounts obtained by performing principal-component analysis on the fluorescence spectrum, a first component factor (i component) and a second component factor (k component) shown in Fig. 4 are used. However, as long as the obtained feature amounts of the fluorescence spectrum of the dialysis drainage can be classified, other component factors obtained by principal-component analysis may be used instead of the first component factor and the second component factor.

The concentration calculation device 16 further includes the calibration model creation unit 93 that creates calibration models Ma to Md by performing multivariate analysis on the fluorescence spectrum for each of the plurality of groups A to D. Therefore, it is possible to simply create the calibration models Ma to Md with high reliability.

Since the multivariate analysis is any of partial least squares (PLS) regression analysis, principal component regression analysis, multiple regression analysis, support vector machine regression analysis, and machine learning analysis, calibration models Ma to Md can be simply and accurately created.

Since the concentration calculation device 16 includes the storage unit 91 that stores a plurality of albumin concentrations calculated at a plurality of time points after the dialysis treatment starts, for example, various numerical values can be computed using the calculated albumin concentration, and the status of the dialysis treatment can be analyzed.

The concentration calculation device 16 includes the integration value calculation unit 100 that calculates a time integration value of the product of albumin concentrations corresponding to a plurality of time points stored in the storage unit 91 and the dialysis drainage flow rate after the dialysis treatment starts. Since the integration value indicates an albumin leakage amount at that time, the albumin leakage amount in the dialysis treatment can be compared with a preset allowable albumin leakage amount, and it is possible to determine the appropriateness of the albumin leakage amount.

The concentration calculation device 16 includes the change rate calculation unit 101 that calculates a rate of change in the albumin concentrations corresponding to a plurality of time points stored in the storage unit 91. Therefore, for example, as in the example A in which the absolute value of the rate of change (dC/dt) in albumin with respect to time in the initial stage shown in Fig. 8 is large and fluctuations quickly fall to a low value, it is possible to determine that the hollow fiber membrane is clogged rapidly during the dialysis treatment.

The concentration calculation device 16 includes the difference calculation unit 102 that calculates a difference between the albumin concentrations corresponding to a plurality of time points stored in the storage unit 91 and a predetermined value. Therefore, for example, as shown in Fig. 9, when the difference between the calculated concentration and the predetermined value is large, it is possible to understand that the albumin leakage amount is larger than expected.

The concentration calculation device 16 includes the estimated value calculation unit 103 that calculates a formula of the albumin concentration over time after the dialysis treatment starts based on albumin concentrations corresponding to a plurality of time points stored in the storage unit 91, and calculates an estimated value of the total amount of albumin in the dialysis drainage from when the dialysis treatment starts until the dialysis treatment ends based on the formula over time. Therefore, an estimated total leakage amount of albumin in the dialysis treatment can be compared with a preset total allowable albumin leakage amount, and it is possible to determine the appropriateness of the dialysis treatment.

Since the concentration calculation device 16 includes the display unit 73 that displays information about the albumin concentration calculated by the concentration calculation unit 72, a user can determine the albumin concentration in the dialysis treatment in real time.

In the dialysis drainage to which excitation light is emitted, the flow rate is a continuous flow of 10 mL/min or more and 1,000 mL/min or less. In the clinical field of dialysis treatment, there are many types of equipment such as dialysis devices and beds, and there is almost no space in which to install an optical monitor. Therefore, the optical monitor preferably does not require a component in which the dialysis drainage is stored, a separation component such as a membrane and chromatography, or a component that captures a part of the dialysis drainage with a pump. Therefore, it is preferable to connect the optical monitor 70 to the drainage circuit 13 in a direct or branched manner and measure with a continuous flow, and the flow rate is preferably 10 mL/min or more and 1,000 mL/min or less.

In the above embodiment, the classification unit 71 divides a plurality of groups A to D based on the feature amount obtained by performing principal-component analysis on the fluorescence spectrum acquired from a detector 81. However, as the classification method, in addition to the principal-component analysis, a classification method such as factor analysis, discriminant analysis, cluster analysis, correspondence analysis, KNN analysis, SIMCA analysis, and PLS-DA may be used. In addition, the concentration calculation device 16 may include a group creation unit that creates a plurality of groups related to the fluorescence spectrums that affect concentration calculation.

In the above embodiment, in the classification unit 71, for a predetermined element that affects concentration calculation, the spectrums of fluorescence detected by the detection unit 81 are classified into one of a plurality of predetermined groups, but the predetermined element that affects concentration calculation is not limited to the fluorescence spectrum, and may be another element or may include another element. For example, the predetermined element that affects concentration calculation may be any of a fluorescence spectrum, a time elapsed from when the dialysis treatment starts, a patient to be treated with dialysis, a patient treatment method, a type of a filter of a dialyzer used in the dialysis treatment, the intensity of fluorescence detected by the detection unit 81, or a maximum value of the fluorescence spectrum in a predetermined wavelength range, or one or more setting parameters for detection of the detection unit 81. In this case, the plurality of groups classified by the classification unit 71 may be divided based on, in addition to the fluorescence spectrum, any of a time elapsed from when the dialysis treatment starts, a patient to be treated with dialysis, a patient treatment method, a type of a filter of a dialyzer used in the dialysis treatment, the intensity of fluorescence detected by the detection unit 81, or a maximum value of the fluorescence spectrum in a predetermined wavelength range, or one or more setting parameters for detection of the detection unit 81.

For example, regarding the time elapsed from when the dialysis treatment starts, for example, the dialysis treatment time is divided into four time blocks of 0 to 20 minutes, 20 to 60 minutes, 60 to 120 minutes, and 120 to 240 minutes, the groups are divided into four groups, and a calibration model corresponding to each group is created. Therefore, the classification unit 71 classifies the elapsed time, which is the predetermined element, into one of four groups each time during the dialysis treatment, and the concentration calculation unit 72 calculates an albumin concentration using the calibration model of the classified group.

Regarding patients to be treated with dialysis, for example, 10 patients are divided into four groups: 2 patients, 2 patients, 3 patients, and 3 patients, and a calibration model corresponding to each group is created. Therefore, the classification unit 71 classifies the patient, which is the predetermined element, into one of four groups during the dialysis treatment, and the concentration calculation unit 72 calculates an albumin concentration using the calibration model of the classified group.

Regarding the patient treatment method, for example, the methods are divided into three groups: hemodialysis, hemodiafiltration (low Alb leakage), and hemodiafiltration (high Alb leakage), and a calibration model corresponding to each group is created. Therefore, the classification unit 71 classifies the treatment method, which is the predetermined element, into one of three groups during the dialysis treatment, and the concentration calculation unit 72 calculates an albumin concentration using the calibration model of the classified group.

Regarding the type of the filter of the dialyzer used in the dialysis treatment, for example, the groups are divided into four groups: a dialyzer, a dialysis filter with almost no albumin leakage, a dialysis filter with a low albumin leakage amount, and a dialysis filter with a large albumin leakage amount, and a calibration model corresponding to each group is created. Therefore, the classification unit 71 classifies the type of the filter, which is the predetermined element, into one of four groups during the dialysis treatment, and the concentration calculation unit 72 calculates an albumin concentration using the calibration model of the classified group.

Regarding the intensity of fluorescence detected by the detection unit 81, for example, the groups are divided into four groups in descending order of the fluorescence intensity, and a calibration model corresponding to each group is created. Therefore, the classification unit 71 classifies the intensity of fluorescence, which is the predetermined element, detected by the detection unit 81 during the dialysis treatment into one of four groups, and the concentration calculation unit 72 calculates an albumin concentration using the calibration model of the classified group.

Regarding the maximum value of the fluorescence spectrum in a predetermined wavelength range, for example, 380 nm or more and 480 nm or less, for example, the groups are divided into four groups in descending order of the maximum value, and a calibration model corresponding to each group is created. Therefore, the classification unit 71 classifies the maximum value of the fluorescence spectrum in a predetermined wavelength range, which is the predetermined element, detected by the detection unit 81 during the dialysis treatment into one of four groups, and the concentration calculation unit 72 calculates an albumin concentration using the calibration model of the classified group.

Here, the wavelength range of 380 nm or more and 480 nm or less of fluorescence corresponds to an excitation wavelength at which a fluorescence characteristic of albumin exhibits a sub-peak, and is a range highly correlated with the albumin concentration.

Regarding the setting parameters for detection by the detection unit 81, for example, the integration time (corresponding to the camera exposure time), which is a setting parameter, is divided into four groups, and a calibration model corresponding to each group is created. Therefore, the classification unit 71 classifies the integration time for the detection unit, which is the predetermined element, detected by the detection unit 81 during the dialysis treatment into one of four groups, and the concentration calculation unit 72 calculates an albumin concentration using the calibration model of the classified group. In particular, since the fluorescence intensity is low in a low concentration area (about 1 mg/dL or less), the fluorescence intensity can increase when the integration time, which is a setting parameter of the detection unit, is made longer. However, in a high concentration area (about 30 mg/dL or more), if the integration time is similarly made longer, the upper limit of the detection value of the detector may be exceeded. Therefore, it is necessary to automatically switch the integration time depending on the concentration, actually, the fluorescence intensity, the elapsed dialysis time, and the like. Therefore, classification into groups is performed according to each integration time, which is a setting parameter, a calibration model is created for each group, and thus the accuracy of concentration calculation can be improved.

Among the above predetermined elements that affect concentration calculation, for example, classification of the patient to be treated with dialysis, the patient treatment methods, and the type of the filter of the dialyzer used in the dialysis treatment is performed using required information input from the input unit 74 by a user.

Incidentally, the albumin concentration calculated in real time during the dialysis treatment for actual (clinical) patients may have, for example, an error due to various factors derived from a living body. Assuming such a case, it is necessary to determine whether the calculated albumin concentration is allowable (used) as an appropriate value. In this case, parameter values related to concentration computation may be set so that the relationship between the %error A of the albumin concentration allowed in any time block of the first half of the dialysis treatment and the %error B of the measured concentration allowed in any time block of the second half of the dialysis treatment is $A \leq B$.

For example, the estimated difference E shown in Fig. 12 is set as an error. That is, the estimation curve of the albumin concentration represented by Formula (1) is obtained using at least 3 or more points of the real time $t_n$ of the concentration of albumin in the dialysis drainage up to the calculation point of the albumin concentration at the previous time $t_{n-1}$. Fig. 13 shows the estimation curve of albumin concentrations at 5 points. Therefore, if the estimated difference rate $EF(t_n)$ represented by Formula (4) obtained by dividing the estimated difference $E(t_n)$ defined by Formula (2) from the estimated albumin concentration $C(t_n)$ in Formula (1) by the root mean square $RMSD(t_{n-1})$ of the estimated difference $E(t_{n-1})$ to the real time $t_{n-1}$ represented by Formula (3) satisfies $-1.0 \leq EF(t_n) \leq 1.0$ at t<a dialysis time of 60 minutes, satisfies $-1.2 \leq EF(t_n) \leq 1.2$ at a dialysis time of 60 minutes≤t<a dialysis time of 120 minutes, and satisfies $-1.5 \leq EF(t_n) \leq 1.5$ at a dialysis time of 120 minutes≤t, the albumin concentration calculated at the real time $t_n$ is used.

$$C(t)=A\times t\hat{}(-B)\cdots(1)$$

$$E(t_n)=(\text{calculated concentration at real time } t_n)-C(t_n)\cdots(2)$$

$$RMSD(t_{n-1})=\{[\Sigma(E(t_i)\hat{}2)]/(n-1)\}\hat{}0.5\cdots(3)$$

$$EF(t_n)=E(t_n)/RMSD(t_{n-1})\cdots(4)$$

C: estimated concentration of albumin in the dialysis drainage [mg/dL], A: constant, t: time [min], B: constant, E: estimated difference, subscript i: i-th calculated concentration, subscript n: n-th calculated concentration.

In Fig. 12, since the calculated concentration at the sixth point is at a dialysis time of 40 minutes (t=40), it is used when the EF value determined from the estimated difference E of the calculated concentration is -1.0 or more and 1.0 or less.

As shown in Fig. 13, in the curve of the change of the concentration of albumin in the dialysis drainage, in the case of the dialysis treatment for 4 hours, it can be said that a total amount of about 80% of albumin in the dialysis drainage is discharged in 1 hour after the dialysis treatment starts. In addition, it can be understood that the initial concentration of albumin in dialysis is about 20 times the final concentration in dialysis or more. Therefore, for example, when a total albumin leakage amount is determined from the calculated albumin concentration, if the %error from the second half to the end of the dialysis treatment is set to be larger than the %error at the beginning of the dialysis treatment, there is no large error in the total albumin leakage amount. On the other hand, generally, if the concentration calculation target has a lower concentration, in order to measure the concentration with higher accuracy, a more expensive optical monitor or the like tends to be required, but it is possible to design a concentration calculation device at a lower cost based on the concept of the above allowable error.

While preferable embodiments of the present invention have been described above with reference to the appended drawings, the present invention is not limited thereto. It can be clearly understood that those skilled in the art can realize various modifications or alternations within the scope of ideas described in the scope of the claims, and these are naturally also included in the technical scope of the present invention.

For example, in the above embodiments, the concentration calculation target to which excitation light is emitted is dialysis drainage containing albumin (protein solution), and the concentration calculation device 16 calculates a concentration of albumin contained in the dialysis drainage, but the present invention can also be applied for calculation of the concentration of a substance other than albumin (for example, proteins) in the dialysis drainage. In addition, in the present invention, excitation light is emitted to a concentration calculation target other than the dialysis drainage, and a substance contained in the concentration calculation target may be used for calculation.

In addition, the configuration of the dialysis system 1 is not limited to one in which hemodiafiltration is performed as in the above embodiment. For example, when hemodialysis is performed in the dialysis system 1, the fluid substitution pump 50 or the fluid substitution circuit 14 may not be used. The fluid substitution circuit 14 may be connected to the blood return line 33 instead of the blood removal line 31. In addition, the present invention can be applied to not only the dialysis system but also a blood treatment system that performs other blood treatment operations. For example, the present invention can be applied to a blood treatment system that performs plasma exchange therapy, leukocyte removal therapy, continuous renal replacement therapy, and the like.

(Examples)

(Example 1)

Online hemodiafiltration therapy with a pre-replacement solution volume of 45 L was performed on dialysis patients with a dialysis history of 4 years using an MFX-21M eco (commercially available from Nipro Corporation) filter. Fig. 14 shows the fluorescence spectrum obtained by emitting UV rays to the dialysis drainage by the optical monitor in this case. Fig. 15 shows the albumin concentration (calculated concentration) calculated by the method of the above concentration calculation device 16 from the obtained fluorescence spectrum. Fig. 16 shows classification of the groups A to D used when the albumin concentration was calculated. This group classification is performed based on the feature amount obtained by performing principal-component analysis on the fluorescence spectrum. Specifically, first, fluorescence spectrums of 76 dialysis drainage samples obtained from a plurality of dialysis patients detected by the optical monitor shown in Fig. 14 were subjected to principal-component analysis. Each fluorescence spectrum was obtained as a 51-dimensional vector. According to principal-component analysis, an eigenmatrix to be converted into a first component factor, a second component factor, ···a $52^{nd}$ component factor was obtained and factor scores were obtained from the measured fluorescence spectrum and the eigenmatrix. A graph shown in Fig. 16 with a horizontal axis that represents the first component which is a component factor indicating the most characteristic feature of the fluorescence spectrum and a vertical axis that represents the second component indicating the next characteristic feature of the fluorescence spectrum was created, and the components were divided into four groups A to D. Then, a calibration model for each of the groups A to D was created from the fluorescence spectrum of the sample corresponding to each of the groups A to D. The fluorescence spectrum obtained from the optical monitor was classified into one of the four groups A to D and the albumin concentration was calculated using the calibration model corresponding to the classified group. Fig. 15 shows the calculated concentration and the measured concentration. It was found that the calculated concentration and the measured concentration were favorably matched. It was confirmed that the concentration was accurately calculated even in the clinical dialysis drainage in which various substances derived from a living body were mixed.

(Example 2)

Online hemodiafiltration therapy with a pre-replacement solution volume of 16L was performed on dialysis patients with a dialysis history of 4 years using an ABH-21P (commercially available from Asahi Kasei Medical Co., Ltd.) filter. UV rays were emitted to the dialysis drainage and a fluorescence spectrum was obtained. The obtained fluorescence spectrum was classified into one of the plurality of groups, and the albumin concentration was calculated from the calibration model of the corresponding group. Group classification was performed using principal-component analysis as in Example 1. The estimated total albumin leakage amount estimated using Formula (1) from the albumin concentration calculated up to 60 minutes in the method of the estimated value calculation unit 103 described above was 1.3 g. On the other hand, the actual total albumin leakage amount obtained from examination data was 1.2 g, and it was found that the estimated total albumin leakage amount and the actual total albumin leakage amount were favorably matched. It was confirmed that the estimated total albumin leakage amount obtained from the concentration calculation device of the present invention accurately matched the measured value.

(1) Feature amount obtained by performing principal-component analysis on spectrum

When one calibration model was created and verified without classifying the 76 dialysis drainage samples in Example 1 into groups, the correlation coefficient (R) and the root mean square error (RMSE) of 19 samples of verification data were 0.729 and 3.77 mg/dL, respectively. On the other hand, when classification (Example 1) into groups was performed according to principal-component analysis, each calibration model was created, and verification was performed with verification data, the correlation coefficient (R) and the root mean square error (RMSE) were 0.972 and 0.966 mg/dL, respectively. The correlation coefficient (R) increased, the root mean square error (RMSE) decreased, and the concentration calculation accuracy was improved.

(2) Time elapsed from when dialysis treatment starts

When one calibration model was created and verified without classifying the 76 dialysis drainage samples in Example 1 into groups, the correlation coefficient (R) and the root mean square error (RMSE) of 19 samples of verification data were 0.729 and 3.77 mg/dL, respectively. On the other hand, when classification into four groups was performed according to the elapsed time (time blocks of 0 to 20 minutes, 20 to 60 minutes, 60 to 120 minutes, and 120 to 240 minutes), each calibration model was created, and verification was performed with verification data, the correlation coefficient (R) and the root mean square error (RMSE) were 0.986 and 0.790 mg/dL, respectively. The correlation coefficient (R) increased, the root mean square error (RMSE) decreased, and the concentration calculation accuracy was improved.

(3) Intensity of fluorescence detected by detection unit

When one calibration model was created and verified without classifying the 76 dialysis drainage samples in Example 1 into groups, the correlation coefficient (R) and the root mean square error (RMSE) of 19 samples of verification data were 0.729 and 3.77 mg/dL, respectively. On the other hand, when the fluorescence intensities were classified into four groups in the order of the fluorescence intensity, each calibration model was created, and verification was performed with verification data, the correlation coefficient (R) and the root mean square error (RMSE) were 0.967 and 1.57 mg/dL, respectively. The correlation coefficient (R) increased, the root mean square error (RMSE) decreased, and the concentration calculation accuracy was improved.

(4) Setting parameters for detection of detection unit

Particularly, in a low concentration area (about 1 mg/dL or less), since the fluorescence intensity was low, if the integration time, which is a setting parameter of the detection unit, was lengthened, it was possible to increase the fluorescence intensity. However, in a high concentration area (about 30 mg/dL or more), if the integration time was similarly made longer, the upper limit of the detection value of the detector may be exceeded. Therefore, it is necessary to automatically switch the integration time depending on the concentration, actually, the fluorescence intensity, the elapsed dialysis time, and the like. Therefore, classification into groups was performed according to the integration time, which is a setting parameter, and a calibration model was created for each group, and thus the accuracy of concentration calculation was improved.

(5) Patient to be treated with dialysis

When one calibration model was created and verified without classifying the 76 dialysis drainage samples in Example 1 into groups, the correlation coefficient (R) and the root mean square error (RMSE) of 19 samples of verification data were 0.729 and 3.77 mg/dL, respectively. On the other hand, when classification into groups was performed for each patient (6 patients), each calibration model was created, and verification was performed with verification data, the correlation coefficient (R) and the root mean square error (RMSE) were 0.888 and 1.90 mg/dL, respectively. The correlation coefficient (R) increased, the root mean square error (RMSE) decreased, and the accuracy of concentration calculation was improved.

(6) Patient treatment method

When one calibration model was created and verified without classifying the 76 dialysis drainage samples in Example 1 into groups, the correlation coefficient (R) and the root mean square error (RMSE) of 19 samples of verification data were 0.729 and 3.77 mg/dL, respectively. On the other hand, when classification into three groups was performed for dialysis treatments (hemodialysis, hemodiafiltration low Alb leakage, hemodiafiltration high Alb leakage), each calibration model was created, and verification was performed with verification data, the correlation coefficient (R) and the root mean square error (RMSE) were 0.954 and 1.25 mg/dL, respectively. The correlation coefficient (R) increased, the root mean square error (RMSE) decreased, and the accuracy of concentration calculation was improved.

(7) Type of filter used in dialysis treatment

When one calibration model was created and verified without classifying the 76 dialysis drainage samples in Example 1 into groups, the correlation coefficient (R) and the root mean square error (RMSE) of 19 samples of verification data were 0.729 and 3.77 mg/dL, respectively. On the other hand, when classification into four groups was performed for each filter (4 types), each calibration model was created, and verification was performed with verification data, the correlation coefficient (R) and the root mean square error (RMSE) were 0.981 and 0.880 mg/dL, respectively. The correlation coefficient (R) increased, the root mean square error (RMSE) decreased, and the accuracy of concentration calculation was improved.

Second embodiment

<Dialysis system>

Fig. 17 is an explanatory diagram showing a configuration outline of a dialysis system 1 as a blood treatment system on which a concentration calculation device is mounted according to the present embodiment.

The dialysis system 1 includes, for example, the dialyzer 10, the blood circuit 11, the dialysate circuit 12, the drainage circuit 13, the fluid substitution circuit 14, the control device 15, and the concentration calculation device 16.

The dialyzer 10 is, for example, a hollow fiber module having a built-in hollow fiber membrane, and can separate unnecessary constituents from blood. The dialyzer 10 has a cylindrical container 20, and a plurality of hollow fiber membranes 21 are disposed inside the cylindrical container 20 in a longitudinal direction. The hollow fiber membrane 21 can separate unnecessary constituents from blood. An inlet 22 and an outlet 23 that lead to the space inside the tube (blood side) of the hollow fiber membrane 21 are provided above and below the cylindrical container 20. Two entrances 24 and 25 that lead to the space outside the tube (dialysate side) of the hollow fiber membrane 21 are provided at the side part of the cylindrical container 20.

The blood circuit 11 includes, for example, a blood removal line 31 that connects a blood removal unit 30 and the dialyzer 10, and a blood return line 33 that connects the dialyzer 10 and a blood return unit 32. The blood removal line 31 and the blood return line 33 are mainly composed of a soft tube. The blood removal line 31 is connected to the inlet 22 of the dialyzer 10, and the blood return line 33 is connected to the outlet 23 of the dialyzer 10.

For example, a blood pump 40 is provided at the blood removal line 31. In addition, a drip chamber 41 is connected to the blood removal line 31. The drip chamber 41 may not be provided.

The dialysate circuit 12 is connected to the entrance 25 of the dialyzer 10 from a dialysate supply source (not shown). The drainage circuit 13 is connected to a drainage unit (not shown) from the entrance 24 of the dialyzer 10. A supply/drainage pump (not shown) that supplies a dialysate to the dialyzer 10 through the dialysate circuit 12 and drains a dialysate from the dialyzer 10 through the drainage circuit 13 is provided at the dialysate circuit 12 and the drainage circuit 13.

For example, the fluid substitution circuit 14 is connected to the drip chamber 41 (the blood circuit 11) from the dialysate circuit 12. When the drip chamber 41 is not provided, the fluid substitution circuit 14 is directly connected to the blood removal line 31. A fluid substitution pump 50 is provided at the fluid substitution circuit 14.

The control device 15 is, for example, a computer. For example, when a CPU executes a program stored in a storage unit, operations of the blood pump 40 and the fluid substitution pump 50 are controlled, and dialysis processing for dialysis treatment can be performed. Here, communication between the control device 15 and various devices (the blood pump 40 and the fluid substitution pump 50) may be performed via a wire such as a communication cable, or may be performed in a wireless manner such as through Bluetooth (registered trademark).

In the dialysis treatment, in the blood circuit 11, the patient's blood is sent from the blood removal unit 30 to the space inside the tube of the hollow fiber membrane 21 of the dialyzer 10, passes through the dialyzer 10, and is then returned to the patient from the blood return unit 32. In this case, the dialysate passes through the dialysate circuit 12, is sent to the space outside the tube of the hollow fiber membrane 21 of the dialyzer 10, and then drained through the drainage circuit 13. In the dialyzer 10, mainly unnecessary constituents in the blood flowing through the space inside the tube of the hollow fiber membrane 21 flow out to the space outside the tube (dialysate side) through the hollow fiber membrane 21, and are discharged together with the dialysate. A substitution fluid (dialysate) of the dialysate circuit 12 is supplied to the blood circuit 11 through the fluid substitution circuit 14 and a predetermined component is refilled in the blood. Here, hemodiafiltration in which a substitution fluid is refilled and hemodialysis in which a substitution fluid is not refilled can be performed.

<Concentration calculation device>

The concentration calculation device 16 includes an optical monitor 70 that emits excitation light to dialysis drainage that is an emission target containing albumin (protein solution) and detects fluorescence generated from the dialysis drainage, a concentration calculation unit 71 that calculates a concentration of albumin contained in the dialysis drainage based on fluorescence intensities of a plurality of wavelengths in a predetermined wavelength range of the fluorescence spectrum acquired by the optical monitor 70 and a calibration model provided in advance, and a display unit 72. Here, the optical monitor 70, communication between the concentration calculation unit 71 and the display unit 72 may be performed via a wire such as a communication cable or may be performed in a wireless manner such as through Bluetooth (registered trademark).

For example, as shown in Fig. 18, the optical monitor 70 includes an emission unit 80 that emits excitation light to the dialysis drainage in the pipe of the drainage circuit 13 and a detection unit 81 that detects fluorescence generated from the dialysis drainage.

The emission unit 80 can emit light having a wavelength of 300 nm or more and 400 nm or less, which excites fluorescence exhibiting a sub-peak of albumin shown in Fig. 19. A light source of the emission unit 80 is not particularly limited, and for example, a halogen lamp, a xenon lamp, a deuterium lamp, or an LED may be used. The wavelength of excitation light emitted from the emission unit 80 is preferably 310 nm or more and 380 nm or less, and more preferably 330 nm or more and 350 nm or less. Here, the excitation light emitted from the emission unit 80 may have a continuous wavelength or may have a wavelength of a certain value (for example, 340 nm).

The detection unit 81 detects fluorescence generated from the dialysis drainage to obtain a fluorescence spectrum. The detection unit 81 may detect fluorescence in a wavelength range of 310 nm or more and 850 nm or less. The fluorescence wavelength detected by the detection unit 81 is preferably in a range of a wavelength obtained by adding 10 nm to a wavelength at which fluorescence excites (excitation wavelength) (if there is a range in the excitation wavelength, the lower limit of the excitation wavelength range+10 nm) or more and 850 nm or less, and more preferably in a range of a wavelength obtained by adding 10 nm to the excitation wavelength (if there is a range in the excitation wavelength, the lower limit of the excitation wavelength range+10 nm) or more and 800 nm or less. In addition, for example, the emission unit 80 and the detection unit 81 are disposed in directions perpendicular to each other with respect to the pipe of the drainage circuit 13, and acquire fluorescence at an angle perpendicular to the excitation light. Here, the disposition of the emission unit 80 and the detection unit 81 is not limited thereto.

The concentration calculation unit 71 is a computer. For example, as shown in Fig. 20, the concentration calculation unit 71 includes the concentration computation unit 90 that calculates a concentration of albumin in the dialysis drainage using information on fluorescence intensities of a plurality of wavelengths in a predetermined wavelength range of the fluorescence spectrum acquired by the optical monitor 70 and the calibration model obtained by multivariate analysis, the storage unit 91 that stores the albumin concentration obtained by the concentration computation unit 90, the numerical value computation unit 92 that computes various numerical values to be described below using the albumin concentration stored in the storage unit 91, and the calibration model creation unit 93 that creates a calibration model in advance.

For example, the calibration model creation unit 93 acquires the fluorescence spectrum of the pseudodialysis drainage having known albumin concentrations and different albumin concentrations from the optical monitor 70, and creates a calibration model M in advance by multivariate analysis. The multivariate analysis for creating the calibration model M may be any of partial least squares (PLS) regression analysis, principal component regression analysis, multiple regression analysis, support vector machine regression analysis, and machine learning analysis. For example, the created calibration model M is stored in the storage unit 91, and is used as a parameter in the computation process performed by the concentration computation unit 90 and the numerical value computation unit 92. Here, the calibration model M obtained in advance may be stored in the storage unit 91 and this may be used.

An example of the calibration model M is shown below. The calibration model M can be expressed as a formula for obtaining a calculated albumin concentration C by multiplying a fluorescence intensity of each wavelength of the obtained fluorescence spectrum by a coefficient and calculating their sum. The calibration model M is specifically expressed as in Formula (6).

$$C=a_1\times I_1+a_2\times I_2+\cdots+a_n\times I_n+K\cdots(6)$$

C: calculated albumin concentration, $a_n$: coefficient, $I_n$: fluorescence intensity, K: constant, and subscript n: a natural number in which each wavelength from which the fluorescence spectrum is acquired is numbered from the shortest (example: if the wavelengths are 300 nm, 310 nm, 320 nm, ···, 400 nm, the subscripts are 1, 2, 3, ···, 11)

The concentration computation unit 90 acquires a fluorescence spectrum of the dialysis drainage from the optical monitor 70 during dialysis treatment in real time, and computes an albumin concentration from fluorescence intensities of a plurality of wavelengths in a predetermined wavelength range of the fluorescence spectrum and the calibration model M created in advance.

The numerical value computation unit 92 computes various numerical values during dialysis treatment in real time by incorporating a value of the albumin concentration stored in the storage unit 91. As shown in Fig. 21, for example, the numerical value computation unit 92 includes the integration value calculation unit 100 that calculates an integration value of the product of the albumin concentration and the dialysis drainage flow rate after the dialysis treatment starts, the change rate calculation unit 101 that calculates a rate of change (dC/dt) in the albumin concentration with respect to time, the difference calculation unit 102 that calculates a difference between the albumin concentration and a predetermined value, and the estimated value calculation unit 103 that calculates an estimated value of a total amount of albumin in the dialysis drainage from when the dialysis treatment starts until the dialysis treatment ends.

As shown in Fig. 22, the integration value calculation unit 100 calculates an albumin leakage amount at that time point from the product of the value of the albumin (Alb) concentration and the dialysis drainage flow rate after the dialysis treatment starts, and calculates an integration value of the albumin leakage amount from a curve of the albumin leakage amount over time. This integration value is an albumin leakage amount (the shaded part in Fig. 22) to that time point from when the dialysis treatment starts.

As shown in Fig. 23, in the change rate calculation unit 101, the slope of a time change curve of the albumin concentration is a rate of change (dC/dt) in albumin over time.

As shown in Fig. 24, a separately predetermined value in the difference calculation unit 102 is, for example, an albumin concentration calculated from a preset allowable albumin leakage amount, and if the albumin concentration exceeds this, it becomes a threshold value indicating excessive albumin leakage. In this case, the difference calculation unit 102 calculates a difference between an albumin concentration and a threshold value thereof.

First, the estimated value calculation unit 103 obtains A and B shown in Formula (1) from at least 3 or more points of the albumin concentration. According to Formula (1), an albumin leakage amount estimation curve shown in Fig. 25 can be obtained. Then, based on Formula (1), an albumin leakage amount after the dialysis treatment starts is estimated, and an estimated value of an albumin total amount (total leakage amount) per treatment during the dialysis treatment is calculated.

$$C(t)=A\times t^\wedge(-B)\cdots(1)$$

C: concentration in dialysis drainage [mg/dL], A: constant, t: time [min], B: constant

The display unit 72 shown in Fig. 20 is, for example, a panel display that displays the albumin concentration calculated by the concentration computation unit 90 and various numerical values obtained by the numerical value computation unit 92, and various warnings. The warnings are issued, for example, when the albumin concentration calculated by the concentration computation unit 90 exceeds a predetermined range, when the integration value of the albumin leakage amount calculated by the integration value calculation unit 100 exceeds a predetermined range, when the rate of change in the albumin concentration calculated by the change rate calculation unit 101 exceeds a predetermined range, when the difference between the albumin concentration calculated by the difference calculation unit 102 and a threshold value thereof exceeds a predetermined range, and when the estimated value of the total leakage amount of albumin per treatment calculated by the estimated value calculation unit 103 exceeds a predetermined range. Here, the control device 15, the concentration calculation unit 71 and the display unit 72 described above may be units and functions realized by the same computer.

Next, operations of the concentration calculation device 16 configured as described above will be described.

The concentration calculation device 16 continuously or intermittently calculates an albumin concentration in the dialysis drainage of the drainage circuit 13 during the dialysis treatment in real time. The dialysis drainage is a continuous flow with a flow rate of 10 mL/min or more and 1,000 mL/min or less.

Specifically, the emission unit 80 of the optical monitor 70 emits excitation light, and the detection unit 81 detects fluorescence generated from the dialysis drainage. The excitation light emitted from the emission unit 80 includes light having a wavelength of 300 nm or more and 400 nm or less, which excites fluorescence exhibiting a sub-peak of albumin. Here, the excitation light may include light having a wavelength of 310 nm or more and 380 nm or less, light having a wavelength of 320 nm or more and 350 nm or less, light having a wavelength of 330 nm or more and 350 nm or less, and light having a wavelength of 340 nm or more and 350 nm or less.

As shown in Fig. 26, the fluorescence spectrum detected by the detection unit 81 may be in a range of a wavelength obtained by adding 10 nm to a wavelength lower limit of excitation light or more and a wavelength obtained by adding 450 nm to a wavelength upper limit of excitation light or less. In addition, it may be in a range of a wavelength obtained by adding 10 nm to a wavelength lower limit of excitation light or more and a wavelength obtained by adding 400 nm to a wavelength upper limit of excitation light or less. The wavelength range of fluorescence detected by the detection unit 81 is, for example, 310 nm or more and 850 nm or less. In the detection unit 81, parameters related to measurement are adjusted based on the numerical value of the intensity maximum value in a wavelength range of 380 nm or more and 480 nm or less of the obtained fluorescence spectrum, and measurement is performed within the measurement upper limit of the sensor of the detection unit 81.

The concentration computation unit 90 of the concentration calculation unit 71 calculates a concentration of albumin contained in the dialysis drainage based on fluorescence intensities of a plurality of wavelengths in a predetermined wavelength range of the spectrum of fluorescence detected by the detection unit 81 and the calibration model M. The concentration computation unit 90 continuously or intermittently calculates the albumin concentration in the dialysis drainage during the dialysis treatment in real time. The albumin concentration calculated by the concentration computation unit 90 is stored in the storage unit 91 each time. For example, the albumin concentration is displayed on the display unit 72 in real time.

Here, the calibration model M is created in advance before the dialysis treatment starts in the calibration model creation unit 93 and stored in the storage unit 91. For example, the calibration model M is created by acquiring fluorescence spectrums of the pseudodialysis drainage with different albumin concentrations from the optical monitor 70 and performing multivariate analysis. For multivariate analysis, for example, partial least squares (PLS) regression analysis, principal component regression analysis, multiple regression analysis, support vector machine regression analysis or machine learning analysis is used.

The numerical value computation unit 92 takes the albumin concentration calculated by the concentration computation unit 90 from the storage unit 91 and computes various numerical values. The integration value calculation unit 100 computes an integration value of the product of the albumin concentration and the dialysis drainage flow rate after the dialysis treatment starts, the change rate calculation unit 101 computes a rate of change in the albumin concentration, the difference calculation unit 102 computes a difference between the albumin concentration and a predetermined value, and the estimated value calculation unit 103 computes an estimated value of the total amount of albumin in the dialysis drainage from when the dialysis treatment starts until the dialysis treatment ends. For example, these values are displayed on the display unit 72. In addition, if the albumin concentration, the integration value of the albumin leakage amount, the rate of change in the albumin concentration, the difference between the albumin concentration and the threshold value, or the estimated amount of the albumin leakage amount per treatment is not within an appropriate range, a warning is displayed on the display unit 72.

According to the present embodiment, the excitation light that is emitted to the dialysis drainage includes light having a wavelength of 300 nm or more and 400 nm or less, which excites fluorescence exhibiting a sub-peak of albumin, and based on fluorescence intensities of a plurality of wavelengths in a predetermined wavelength range of the fluorescence spectrum generated from the dialysis drainage by emitting the excitation light and the calibration model M, the concentration of albumin contained in the dialysis drainage is calculated. As a result, it is possible to calculate the albumin concentration with high accuracy without complicating the device.

In this regard, for detection of a substance using self-fluorescence and calculation of the amount of the substance present in a mixed solution such as dialysis drainage, referring to the description of paragraph 0048 in Patent Document 4, "an emission spectrum of each fluorescent dye molecule of interest in pathological analysis largely overlaps for a specified emission wavelength, and is provided in the same UV region. In addition to this inconvenience, since the fluorescence spectrum of the molecule described above is relatively wide, it is difficult to deconvolute the measured spectrum, or even if deconvolution is performed, there is a large measurement error. Therefore, based on general fluorescence spectroscopy, it is not possible to accurately determine a concentration of each substance in the dialysate or accurately determine a ratio thereof. That is, it is not possible to quantitatively specify the concentration with high reliability" is generally considered. This has also been confirmed based on the studies performed by the inventors, and in a mixed solution, it is not possible to exclude the influence of contaminants in the examination in the vicinity of a typical excitation wavelength of 280 nm to 296 nm that proteins such as albumin have. Therefore, an excitation wavelength of 300 nm or more and 400 nm or less that is not easily influenced by contaminants is found, and multivariate analysis is then performed on the fluorescence spectrum of a fluorescence wavelength of 310 nm or more and 850 nm or less obtained at an excitation wavelength of 300 nm or more and 400 nm or less, and a calibration model is obtained. Based on the calibration model, the albumin concentration is calculated from the newly obtained fluorescence spectrum, and as a result, it is possible to calculate the albumin concentration with high accuracy.

When the excitation light includes light having a wavelength of 310 nm or more and 380 nm or less, it is possible to calculate the albumin concentration with higher accuracy.

Fig. 27 shows experimental examples of the correlation coefficient R between the calculated albumin concentration and the actual albumin concentration and the root mean square error (RMSE) when the wavelength range in the fluorescence spectrum is changed. The excitation wavelength includes a wavelength of 300 nm or more and 400 nm or less. When the fluorescence start wavelength (minimum wavelength) is set to 310 nm, the error (RMSE) is small. When the fluorescence start wavelength (minimum wavelength) is changed from 310 nm to 340 nm, the error (RMSE) becomes smaller, but the error becomes slightly larger at 350 nm. When the fluorescence end wavelength (maximum wavelength) is changed from 850 nm to 750 nm, the error has become smaller. If the fluorescence wavelength is set to be within a range from a wavelength obtained by adding 10 nm to a wavelength of excitation light to a wavelength obtained by adding 450 nm to a wavelength of excitation light, it is possible to realize the albumin concentration with high accuracy. In addition, if the fluorescence wavelength is set to be within a range from a wavelength obtained by adding 10 nm to a wavelength of excitation light to a wavelength obtained by adding 400 nm to a wavelength of excitation light, it is possible to calculate the albumin concentration with high accuracy.

The concentration calculation device 16 further includes the calibration model creation unit 93 that creates the calibration model M by performing multivariate analysis. Therefore, it is possible to simply create the calibration model M with high reliability.

Since the multivariate analysis is any of partial least squares (PLS) regression analysis, principal component regression analysis, multiple regression analysis, support vector machine regression analysis, and machine learning analysis, the calibration model M can be simply and accurately created.

Since the concentration calculation device 16 further includes the storage unit 91 that stores a plurality of albumin concentrations calculated after the dialysis treatment starts, for example, various numerical values can be computed using the calculated albumin concentration, and the status of the dialysis treatment can be analyzed.

The concentration calculation device 16 includes the integration value calculation unit 100 that calculates an integration value of the product of the albumin concentration stored in the storage unit 91 and the dialysis drainage flow rate after the dialysis treatment starts. Since the integration value indicates an albumin leakage amount up to that time, the albumin leakage amount in the dialysis treatment can be compared with a preset allowable albumin leakage amount, and it is possible to determine the appropriateness of the albumin leakage amount.

The concentration calculation device 16 includes the change rate calculation unit 101 that calculates a rate of change (dC/dt) in the albumin concentration stored in the storage unit 91 with respect to time. Therefore, for example, as in the example A in which the absolute value of the rate of change (dC/dt), which is a slope of the curve of albumin that varies over time shown in Fig. 23 is large, it is possible to determine that the hollow fiber membrane is clogged rapidly during the dialysis treatment.

The concentration calculation device 16 includes the difference calculation unit 102 that calculates a difference between the albumin concentration stored in the storage unit 91 and a predetermined value. Therefore, for example, as shown in Fig. 24, when the difference between the calculated concentration and the predetermined value is large, it is possible to understand that the albumin leakage amount is larger than expected.

The concentration calculation device 16 includes the estimated value calculation unit 103 that calculates a formula of the albumin concentration over time after the dialysis treatment starts based on the albumin concentration stored in the storage unit 91 and calculates an estimated value of the total amount of albumin in the dialysis drainage from when the dialysis treatment starts until the dialysis treatment ends based on the formula over time. Therefore, an estimated total leakage amount of albumin in the dialysis treatment can be compared with a preset total allowable albumin leakage amount, and it is possible to determine the appropriateness of the dialysis treatment.

Since the concentration calculation device 16 includes the display unit 72 that displays information about the albumin concentration calculated by the concentration calculation unit 71, a user can determine the albumin concentration in the dialysis drainage during the dialysis treatment in real time.

In the dialysis drainage to which excitation light is emitted, the flow rate is a continuous flow of 10 mL/min or more and 1,000 mL/min or less. In the clinical field of dialysis treatment, there are many types of equipment such as dialysis devices and beds, and there is almost no space in which to install an optical monitor. Therefore, the optical monitor preferably does not require a component in which the dialysis drainage is stored, a separation component such as a membrane and chromatography, or a component that captures a part of the dialysis drainage with a pump. Therefore, it is preferable to connect the optical monitor 70 to the drainage circuit 13 in a direct or branched manner and measure with a continuous flow, and the flow rate is preferably 10 mL/min or more and 1,000 mL/min or less.

Incidentally, the concentration of albumin in the dialysis drainage calculated in real time during the dialysis treatment for actual (clinical) patients may have, for example, an error due to various factors derived from a living body. Assuming such a case, it is necessary to determine whether the calculated albumin concentration is allowable (used) as an appropriate value. In this case, parameter values related to concentration computation may be set so that the relationship between the %error A of the albumin concentration allowed in any time block of the first half of the dialysis treatment and the %error B of the measured concentration allowed in any time block of the second half of the dialysis treatment is A≤B.

For example, the estimated difference E shown in Fig. 28 is set as an error. That is, the estimation curve of the albumin concentration represented by Formula (1) is obtained using at least 3 or more points of the real time $t_n$ of the concentration of albumin in the dialysis drainage up to the calculation point of the albumin concentration at the previous time $t_{n-1}$. Fig. 28 shows the estimation curve of albumin concentrations at 5 points. Therefore, if the estimated difference rate $EF(t_n)$ represented by Formula (4) obtained by dividing the estimated difference $E(t_n)$ defined by Formula (2) from the estimated albumin concentration $C(t_n)$ in Formula (1) by the root mean square $RMSD(t_{n-1})$ of the estimated difference $E(t_{n-1})$ to the real time $t_{n-1}$ represented by Formula (3) satisfies $-1.0 \leq EF(t_n) \leq 1.0$ at t<a dialysis time of 60 minutes, satisfies $-1.2 \leq EF(t_n) \leq 1.2$ at a dialysis time of 60 minutes≤t<a dialysis time of 120 minutes, and satisfies $-1.5 \leq EF(t_n) \leq 1.5$ at a dialysis time of 120 minutes≤t, the albumin concentration calculated at the real time $t_n$ is used.

$$C(t)=A\times t^{\wedge}(-B)\cdots(1)$$

$$E(t_n)=(\text{calculated concentration at real time } t_n)-C(t_n)\cdots(2)$$

$$RMSD(t_{n-1})=\{[\Sigma(E(t_i)^{\wedge}2)]/(n-1)\}^{\wedge}0.5\cdots(3)$$

$$EF(t_n)=E(t_n)/RMSD(t_{n-1})\cdots(4)$$

C: estimated concentration of albumin in dialysis drainage [mg/dL], A: constant, t: time [min], B: constant, E: estimated difference, subscript i: i-th calculated concentration, subscript n: n-th calculated concentration.

In Fig. 28, since the calculated concentration at the sixth point is at a dialysis time of 40 minutes (t=40), it is used when the EF value determined from the estimated difference E of the calculated concentration is -1.0 or more and 1.0 or less.

As shown in Fig. 29, in the curve of the change of the concentration of albumin in the dialysis drainage, in the case of the dialysis treatment for 4 hours, it can be said that a total amount of about 80% of albumin in the dialysis drainage is discharged in 1 hour after the dialysis treatment starts. In addition, it can be understood that the initial concentration of albumin in dialysis is about 20 times the final concentration in dialysis or more. Therefore, for example, when a total albumin leakage amount is determined from the calculated albumin concentration, if the %error from the second half to the end of the dialysis treatment is set to be larger than the %error at the beginning of the dialysis treatment, there is no large error in the total albumin leakage amount. On the other hand, generally, if the concentration of the target is lower, in order to measure the concentration with higher accuracy, a more expensive optical monitor or the like tends to be required, but it is possible to design a concentration calculation device at a lower cost based on the concept of the above allowable error.

While preferable embodiments of the present invention have been described above with reference to the appended drawings, the present invention is not limited thereto. It can be clearly understood that those skilled in the art can realize various modifications or alternations within the scope of ideas described in the scope of the claims, and these are naturally also included in the technical scope of the present invention.

For example, in the above embodiments, the emission target to which excitation light is emitted is dialysis drainage containing albumin, and the concentration calculation device 16 calculates a concentration of albumin contained in the dialysis drainage, but in the present invention, excitation light is emitted to another emission target as long as it contains albumin, and the albumin concentration of the emission target may be calculated.

In addition, the configuration of the dialysis system 1 is not limited to one in which the hemodiafiltration is performed as in the above embodiment. For example, when hemodialysis is performed in the dialysis system 1, the fluid substitution pump 50 or the fluid substitution circuit 14 may not be used. The fluid substitution circuit 14 may be connected to the blood return line 33 instead of the blood removal line 31. In addition, the present invention can be applied not only to a dialysis system but also a blood treatment system that performs other blood treatment operations. For example, the present invention can be applied to a blood treatment system that performs plasma exchange therapy, leukocyte removal therapy, continuous renal replacement therapy, and the like.

(Examples)

(Example 1)

(Albumin concentration calculation example)

The following Table 1 shows compositions of the pseudodialysate. A phosphate buffer (pH 7.4) was used as a solvent.

[Table 1]

Table 1 Pseudodialysis drainage

| Small-molecular-w eight substance | Aromatic amino acid | Low-molecular-wei ght protein | Albumin |
|---|---|---|---|
| Urea<br>Uric acid | Tyrosine<br>Phenylalanine<br>Tryptophan | Lysozyme | Human serum albumin |

The prepared pseudodialysis drainage was flowed through a monitor at a flow rate of 600 mL/min, and an LED emitted light having a peak wavelength of 340 nm (full width of half maximum of 10 nm) to acquire a fluorescence spectrum. Fig. 30 shows an example of the obtained fluorescence spectrum. CA12880MA (commercially available from Hamamatsu Photonics K.K.) was used as the detector having acquired fluorescence. The output of the LED was set to 350 mA, and the integration time of the detector was set to 0.5 s so that the maximum value of the fluorescence spectrum of 380 nm to 480 nm was within the measurement upper limit of the sensor of the detection unit. A calibration model was created in advance from the obtained fluorescence spectrum. From the calibration model, using the pseudodialysis drainage with a known albumin concentration, the albumin concentration was calculated by the concentration calculation device of the present invention, the conformity between the estimated concentration and the actual concentration was verified, and the accuracy of the calibration model was verified. The results are shown in Fig. 31. As a result, the albumin concentration was calculated with high accuracy using the calibration model.

(Comparative Examples 1 and 2)

Using the pseudodialysis drainage prepared in the same manner as in Example 1, using a spectrophotofluorometer (FP-8500: commercially available from JASCO Corporation), excitation light of 280 nm (Comparative Example 1) and 410 nm (Comparative Example 2) was emitted to the pseudodialysis drainage to acquire a fluorescence spectrum. A calibration model was created in advance from the obtained fluorescence spectrum. From the calibration model, using the pseudodialysis drainage with a known albumin concentration, the albumin concentration was calculated in the same manner as in the concentration calculation device of the present invention except for the wavelength of excitation light, the conformity between the estimated concentration and the actual concentration was verified, and the accuracy of the calibration model was verified. Fig. 32 shows the results when the excitation light has an excitation wavelength of 280 nm. Based on the results, it was found that, even with the same actual concentration (known concentration), the estimated concentration varied greatly, and it was difficult to calculate the albumin concentration with high accuracy.

(Examples 2 to 7)

In the same manner as in Comparative Examples 1 and 2, excitation light having a wavelength of 310 nm (Example 2), 320 nm (Example 3), 330 nm (Example 4), 340 nm (Example 5), 350 nm (Example 6), and 380 nm (Example 7) was emitted to the pseudodialysis drainage to obtain a fluorescence spectrum. A calibration model was created in advance from the obtained fluorescence spectrum. From the calibration model, using the pseudodialysis drainage with a known albumin concentration, the albumin concentration was calculated by the concentration calculation device of the present invention, the conformity between the estimated concentration and the actual concentration was verified, and the accuracy of the calibration model was verified. The root mean square error (RMSE) obtained from the difference between the actual concentration and the estimated concentration in this case was determined from the following Formula (5).

$$RMSE=\{[\Sigma((Ce_i-Ck_i)^2)]/n\}^{0.5} \cdots (5)$$

RMSE: root mean square error, Ce: estimated concentration, Ck: actual concentration, subscript i: i-th sample, n: number of samples.

Fig. 33 shows the relationship between the excitation wavelength and the RMSE obtained from Comparative Examples 1 and 2, and Examples 2 to 7. It was found that, when the excitation wavelength was in a range of 300 nm or more and 400 nm or less, the RMSE was small and the accuracy of the estimated concentration was high.

(Albumin concentration change)

If the response of the concentration calculation device was slow with respect to the change in the albumin concentration, it was not possible to follow a remarkable albumin concentration change at the initial stage of dialysis, and it was not possible to perform monitoring in real time, which is not practical. Therefore, in an examination, using the pseudodialysis drainage prepared in the same manner as in Example 1, the albumin concentration was continuously changed, and the response followability and the practicality of the concentration calculation device were checked. A 750 mL pseudodialysis drainage pool was flowed at a flow rate of 500 mL/min, and monitored with the concentration calculation device. A phosphate buffer was added to the pool at the same flow rate of 500 mL/min, and the pseudodialysis drainage pool was mixed and diluted. The albumin concentration was obtained from the calibration model created in advance, and the albumin concentration change is shown in Fig. 34. It was confirmed that the albumin concentration change was almost the same as in the theoretical curve and the response followability of the concentration calculation device was excellent, and there was no problem in practical use.

Industrial Applicability

The present invention is useful to provide a concentration calculation device that can calculate a substance concentration with high accuracy without complicating the device, and a blood treatment system.

Reference Signs List

1 Dialysis system

16 Concentration calculation device

70 Optical monitor

71 Classification unit

72 Concentration calculation unit

73 Display unit

80 Emission unit

81 Detection unit

91 Storage unit

## Claims

**1.** A concentration calculation device, comprising:

an emission unit that emits excitation light to a concentration calculation target;
a detection unit that detects fluorescence generated from the concentration calculation target;
a classification unit that classifies a predetermined element that affects concentration calculation into any of a plurality of predetermined groups; and
a concentration calculation unit that calculates a concentration of a substance contained in the concentration calculation target on the basis of a calibration model that corresponds to a group of the predetermined element classified by the classification unit among a plurality of calibration models provided in advance corresponding to each of the plurality of groups, and fluorescence intensities of a plurality of wavelengths in a predetermined wavelength range of a fluorescence spectrum detected by the detection unit.

2. The concentration calculation device according to claim 1, wherein the element is a spectrum of the fluorescence detected by the detection unit.

3. The concentration calculation device according to claim 2, wherein the plurality of groups are divided, based on a feature amount obtained by performing principal-component analysis on the fluorescence spectrum.

4. The concentration calculation device according to any one of claims 1 to 3, wherein the concentration calculation target is dialysis drainage discharged in dialysis treatment.

5. The concentration calculation device according to claim 1,

wherein the concentration calculation target is dialysis drainage discharged in dialysis treatment, and
wherein the element is any of a spectrum of the fluorescence, a time elapsed from when the dialysis treatment starts, a patient to be treated with dialysis, a patient treatment method, a type of a filter of a dialyzer used in the dialysis treatment, an intensity of fluorescence detected by the detection unit, a maximum value in a predetermined wavelength range of the fluorescence spectrum, and one or more setting parameters for detection of the detection unit.

6. The concentration calculation device according to claim 4 or 5, wherein the substance is albumin in the dialysis drainage.

7. The concentration calculation device according to any one of claims 4 to 6, wherein parameter values related to concentration computation are set so that a relationship between a %error A of a substance concentration allowed in any time block of a first half of the dialysis treatment and a %error B of the measured concentration allowed in any time block of a second half of the dialysis treatment is $A \leq B$.

8. The concentration calculation device according to any one of claims 4 to 7,
wherein an approximate curve of an estimated substance concentration represented by Formula (1) is obtained using at least 3 or more points of the real time $t_n$ of the concentration of the substance in the dialysis drainage up to the calculation point of the substance concentration at an immediately previous time $t_{n-1}$, and when an estimated difference rate $EF(t_n)$ represented by Formula (4) obtained by dividing an estimated difference $E(t_n)$ defined by Formula (2) from an estimated substance concentration $C(t_n)$ in Formula (1) by the root mean square $RMSD(t_{n-1})$ of an estimated difference $E(t_{n-1})$ to the real time $t_{n-1}$ represented by Formula (3) satisfies $-1.0 \leq EF(t_n) \leq 1.0$ at t<a dialysis time of 60 minutes, satisfies $-1.2 \leq EF(t_n) \leq 1.2$ at a dialysis time of 60 minutes≤t<a dialysis time of 120 minutes, and satisfies $-1.5 \leq EF(t_n) \leq 1.5$ at a dialysis time of 120 minutes≤t, the substance concentration calculated at the real time $t_n$ is used:

$$C(t)=A\times t\^{}(-B)\cdots(1)$$

$$E(t_n)=(\text{calculated concentration at real time } t_n)-C(t_n)\cdots(2)$$

$$RMSD(t_{n-1})=\{[\Sigma(E(t_i)\^{}2)]/(n-1)\}\^{}0.5\cdots(3)$$

$$EF(t_n)=E(t_n)/RMSD(t_{n-1})\cdots(4)$$

C: estimated concentration of the substance in the dialysis drainage [mg/dL], A: constant, t: time [min], B: constant, E: estimated difference, subscript i: i-th calculated concentration, subscript n: n-th calculated concentration.

9. The concentration calculation device according to any one of claims 1 to 8, further comprising a calibration model creation unit that creates a plurality of calibration models by performing multivariate analysis on a plurality of fluorescence spectrums corresponding to the plurality of groups respectively.

10. The concentration calculation device according to claim 9, wherein the multivariate analysis is any of partial least squares (PLS) regression analysis, principal component regression analysis, multiple regression analysis, support vector machine regression analysis, and machine learning analysis.

11. The concentration calculation device according to any one of claims 1 to 10, further comprising a storage unit that stores a plurality of substance concentrations calculated by the concentration calculation unit at a plurality of time points.

12. The concentration calculation device according to claim 11, further comprising an integration value calculation unit that calculates a time integration value of a product of each substance concentration corresponding to a plurality of time points stored in the storage unit and a flow rate of the concentration calculation target.

13. The concentration calculation device according to claim 11 or 12, further comprising a change rate calculation unit that calculates a rate of change (dC/dt, C: substance concentration, t: time), with respect to time, in substance concentrations corresponding to a plurality of time points stored in the storage unit.

14. The concentration calculation device according to any one of claims 11 to 13, further comprising a difference calculation unit that calculates a difference between each of the substance concentrations corresponding to a plurality of time points stored in the storage unit and a predetermined value.

15. The concentration calculation device according to any one of claims 11 to 14, further comprising an estimated value calculation unit that calculates a formula of the substance concentration over time, based on each of the substance concentrations corresponding to a plurality of times stored in the storage unit and calculates an estimated value of a total amount of the substance contained in the concentration calculation target, based on the formula over time.

16. The concentration calculation device according to any one of claims 1 to 15, further comprising a display unit that displays information on the substance concentration calculated by the concentration calculation unit.

17. The concentration calculation device according to any one of claims 1 to 16, wherein the concentration calculation target to which the excitation light is emitted is a continuous flow having a flow rate of 10 mL/min or more and 1,000 mL/min or less.

18. A blood treatment system comprising the concentration calculation device according to any one of claims 1 to 17.

19. A concentration calculation device, comprising:

an emission unit that emits excitation light to an emission target containing albumin;
a detection unit that detects fluorescence generated from the emission target; and
a concentration calculation unit that calculates a concentration of albumin contained in the emission target, based on fluorescence intensities of a plurality of wavelengths in a predetermined wavelength range of a fluorescence spectrum detected by the detection unit and a calibration model provided in advance,
wherein the excitation light includes light having a wavelength of 300 nm or more and 400 nm or less, which excites fluorescence exhibiting a sub-peak of the albumin.

20. The concentration calculation device according to claim 19, wherein the excitation light includes light having a wavelength of 310 nm or more and 380 nm or less.

21. The concentration calculation device according to claim 19 or 20, wherein the predetermined wavelength range of the fluorescence spectrum includes a range from a wavelength obtained by adding 10 nm to a wavelength lower limit of the excitation light to a wavelength obtained by adding 450 nm to a wavelength upper limit of the excitation light.

22. The concentration calculation device according to claim 19 or 20, wherein the predetermined wavelength range of the fluorescence spectrum includes a range from a wavelength obtained by adding 10 nm to a wavelength lower limit of the excitation light to a wavelength obtained by adding 400 nm to a wavelength upper limit of the excitation light.

**23.** The concentration calculation device according to any one of claims 19 to 22, further comprising a calibration model creation unit that creates the calibration model by performing multivariate analysis.

**24.** The concentration calculation device according to claim 23, wherein the multivariate analysis is any of partial least squares (PLS) regression analysis, principal component regression analysis, multiple regression analysis, support vector machine regression analysis, and machine learning analysis.

**25.** The concentration calculation device according to any one of claims 19 to 24, wherein the albumin concentration is calculated by formula below:

$$C=a_1\times I_1+a_2\times I_2+\cdots+a_n\times I_n+K$$

C: calculated albumin concentration, $a_n$: coefficient, $I_n$: fluorescence intensity, K: constant, subscript n: a natural number in which each wavelength from which the fluorescence spectrum is acquired is numbered from the shortest.

**26.** The concentration calculation device according to any one of claims 19 to 25, wherein the emission target is dialysis drainage discharged in dialysis treatment.

**27.** The concentration calculation device according to claim 26, further comprising a storage unit that stores a plurality of albumin concentrations calculated by the concentration calculation unit in the dialysis treatment.

**28.** The concentration calculation device according to claim 27, further comprising an integration value calculation unit that calculates an integration value of a product of the albumin concentration stored in the storage unit and a dialysis drainage flow rate after the dialysis treatment starts.

**29.** The concentration calculation device according to claim 27 or 28, further comprising a change rate calculation unit that calculates a rate of change (dC/dt, C: albumin concentration, t: time), with respect to time, in the albumin concentration stored in the storage unit.

**30.** The concentration calculation device according to any one of claims 27 to 29, further comprising a difference calculation unit that calculates a difference between the albumin concentration stored in the storage unit and a predetermined value.

**31.** The concentration calculation device according to any one of claims 27 to 30, further comprising an estimated value calculation unit that calculates a formula of the albumin concentration over time after the dialysis treatment starts, based on the albumin concentration stored in the storage unit, and calculates an estimated value of a total amount of albumin in the dialysis drainage from when the dialysis treatment starts until the dialysis treatment ends, based on the formula over time.

**32.** The concentration calculation device according to any one of claims 26 to 31, further comprising a display unit that displays information on the albumin concentration calculated by the concentration calculation unit.

**33.** The concentration calculation device according to any one of claims 26 to 32, wherein parameter values related to concentration calculation are set so that a relationship between a %error A of an albumin concentration allowed in any time block of a first half of the dialysis treatment and a %error B of the measured concentration allowed in any time block of a second half of the dialysis treatment is $A\leq B$.

**34.** The concentration calculation device according to any one of claims 26 to 33, wherein an approximate curve of the estimated albumin concentration represented by Formula (1) is obtained using at least 3 or more points of the real time $t_n$ of the concentration of albumin in the dialysis drainage up to a calculation point of the albumin concentration at an immediately previous time $t_{n-1}$, and when an estimated difference rate $EF(t_n)$ represented by Formula (4) obtained by dividing an estimated difference $E(t_n)$ defined by Formula (2) from an estimated albumin concentration $C(t_n)$ in Formula (1) by the root mean square $RMSD(t_{n-1})$ of an estimated difference $E(t_{n-1})$ to the real time $t_{n-1}$ represented by Formula (3) satisfies $-1.0\leq EF(t_n)\leq 1.0$ at t<a dialysis time of 60 minutes, satisfies $-1.2\leq EF(t_n)\leq 1.2$ at a dialysis time of 60 minutes≤t<a dialysis time of 120 minutes, and satisfies $-1.5\leq EF(t_n)\leq 1.5$ at a dialysis time of 120 minutes≤t, the albumin concentration calculated at the real time $t_n$ is used:

$$C(t)=A\times t^{\wedge}(-B)\cdots(1)$$

$$E(t_n)=(\text{calculated concentration at real time } t_n)-C(t_n)\cdots(2)$$

$$RMSD(t_{n-1})=\{[\Sigma(E(t_i)^{\wedge}2)]/(n-1)\}^{\wedge}0.5\cdots(3)$$

$$EF(t_n)=E(t_n)/RMSD(t_{n-1})\cdots(4)$$

C: estimated concentration of albumin in the dialysis drainage [mg/dL], A: constant, t: time [min], B: constant, E: estimated difference, subscript i: i-th calculated concentration, subscript n: n-th calculated concentration.

**35.** The concentration calculation device according to any one of claims 26 to 34, wherein the dialysis drainage to which the excitation light is emitted is a continuous flow having a flow rate of 10 mL/min or more and 1,000 mL/min or less.

**36.** A blood treatment system, comprising the concentration calculation device according to any one of claims 19 to 35.

Fig. 1

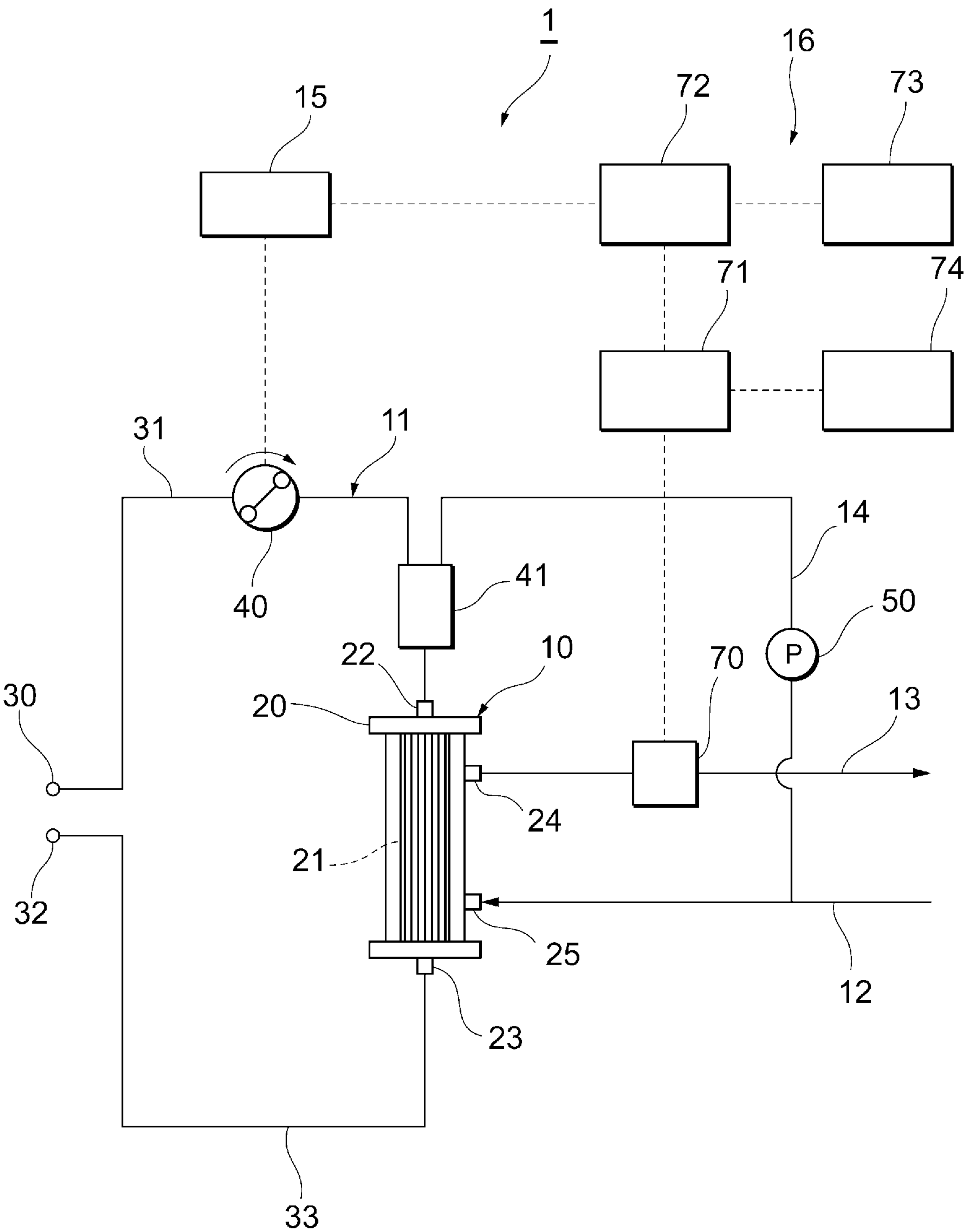
1
15
72
16
73
71
74
31
11
14
50
P
40
41
22
10
70
13
30
20
24
21
32
25
12
23
33

Fig. 2

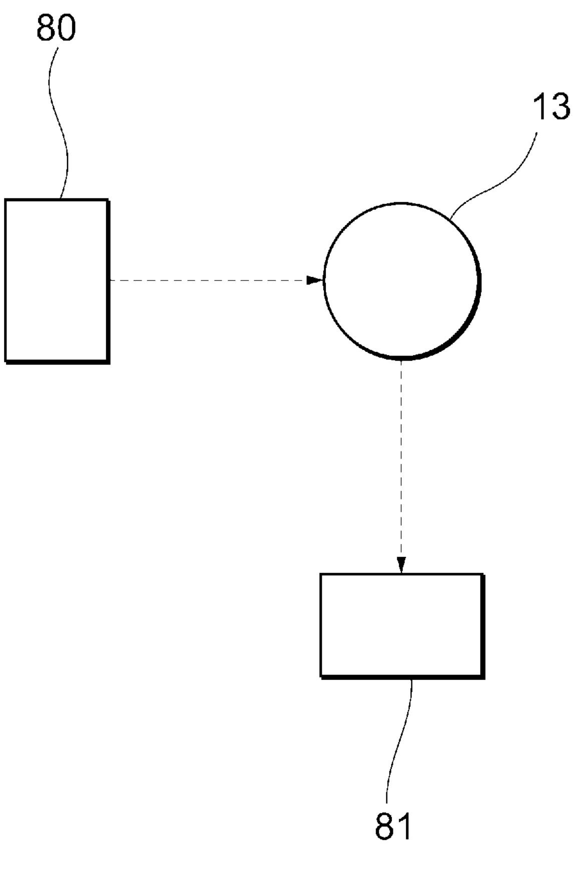

Fig. 3

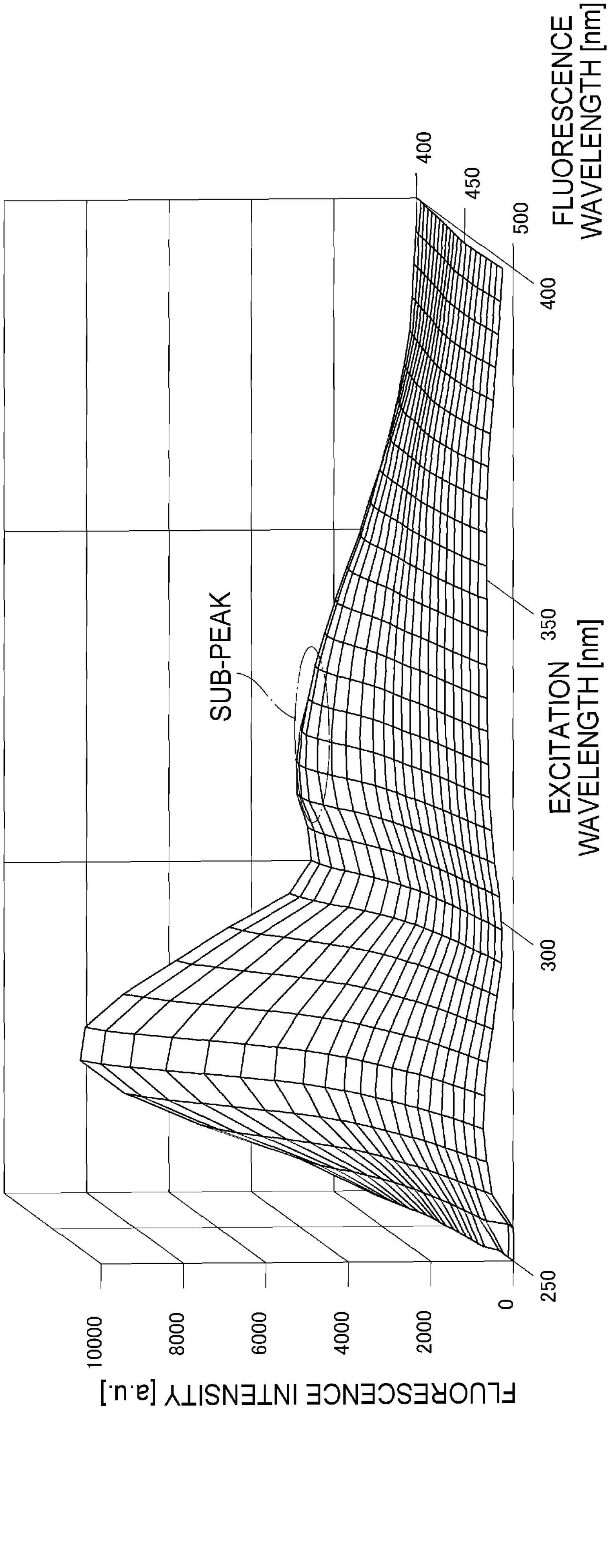
FLUORESCENCE INTENSITY [a.u.]
10000
8000
6000
4000
2000
0
250
300
350
400
400
450
500
SUB-PEAK
EXCITATION WAVELENGTH [nm]
FLUORESCENCE WAVELENGTH [nm]

Fig. 4

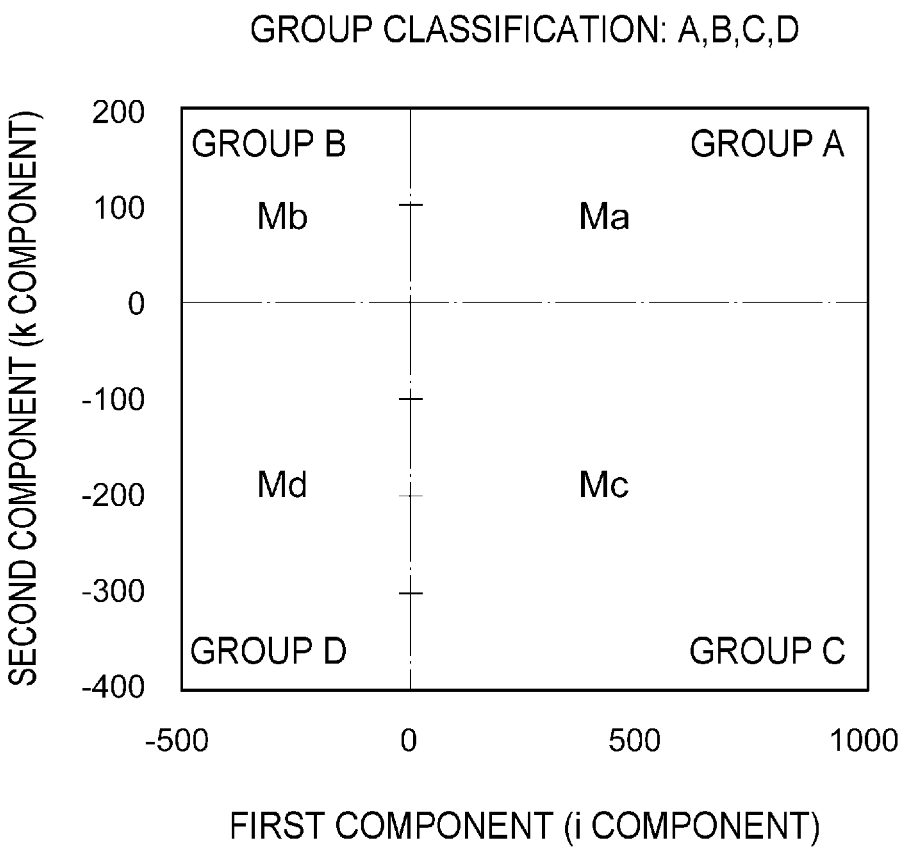
GROUP CLASSIFICATION: A,B,C,D
SECOND COMPONENT (k COMPONENT)
200
100
0
-100
-200
-300
-400
GROUP B
GROUP A
Mb
Ma
Md
Mc
GROUP D
GROUP C
-500
0
500
1000
FIRST COMPONENT (i COMPONENT)

Fig. 5

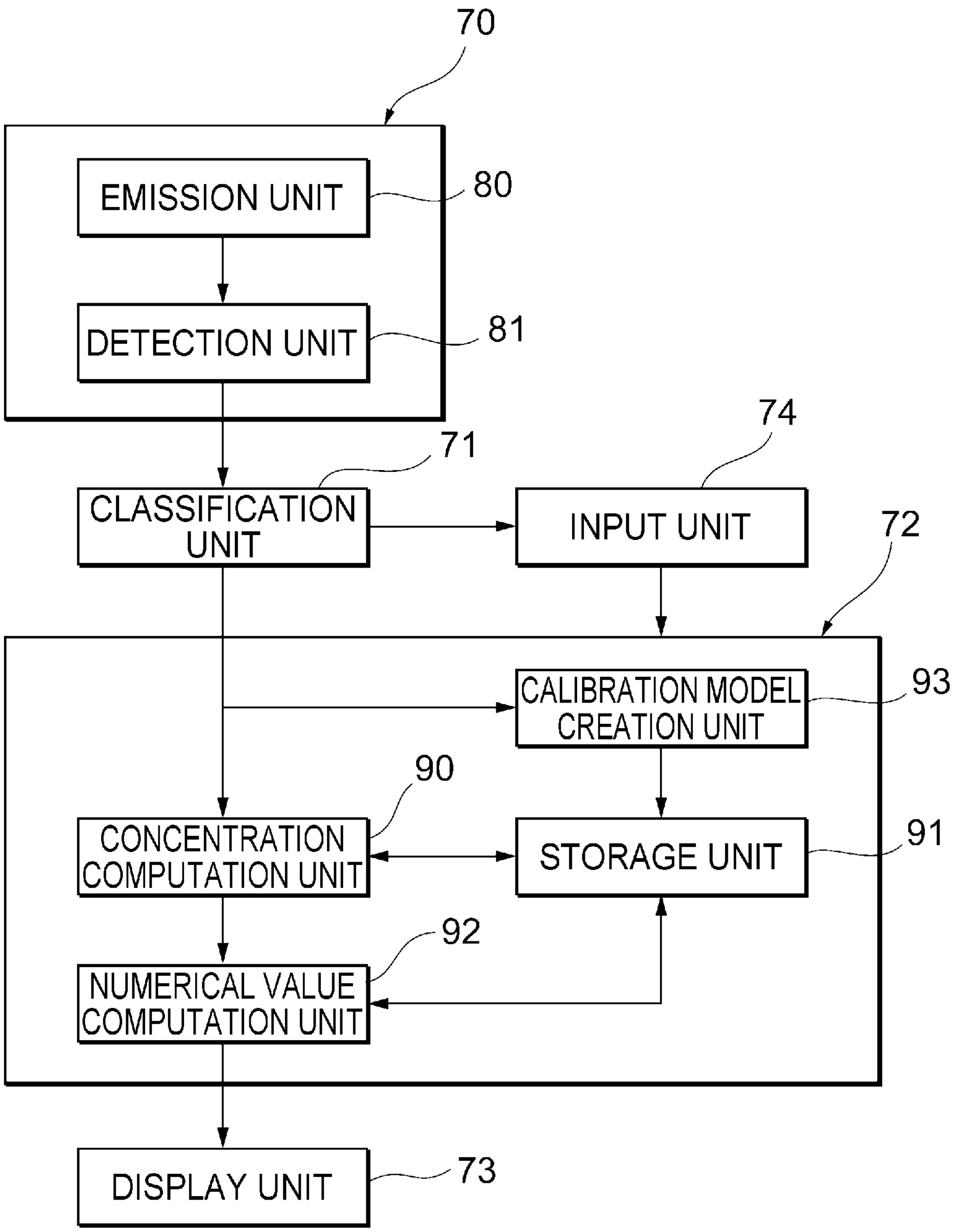
70
EMISSION UNIT
80
DETECTION UNIT
81
71
74
CLASSIFICATION UNIT
INPUT UNIT
72
CALIBRATION MODEL CREATION UNIT
93
90
CONCENTRATION COMPUTATION UNIT
STORAGE UNIT
91
92
NUMERICAL VALUE COMPUTATION UNIT
DISPLAY UNIT
73

Fig. 6

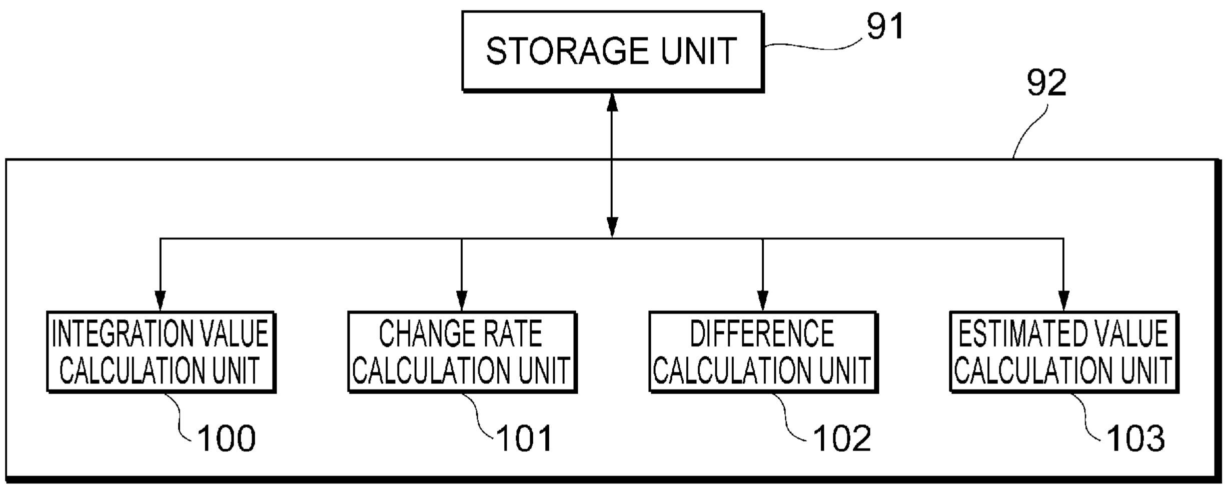
STORAGE UNIT
91
92
INTEGRATION VALUE CALCULATION UNIT
100
CHANGE RATE CALCULATION UNIT
101
DIFFERENCE CALCULATION UNIT
102
ESTIMATED VALUE CALCULATION UNIT
103

Fig. 7

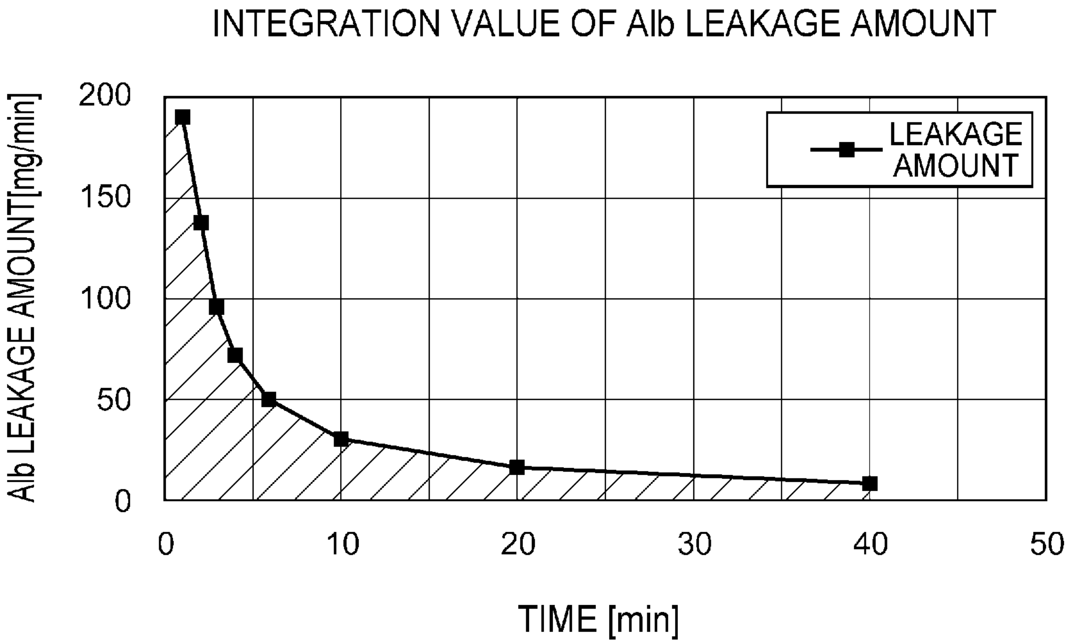
INTEGRATION VALUE OF Alb LEAKAGE AMOUNT
Alb LEAKAGE AMOUNT[mg/min]
200
150
100
50
0
0
10
20
30
40
50
TIME [min]
LEAKAGE AMOUNT

Fig. 8

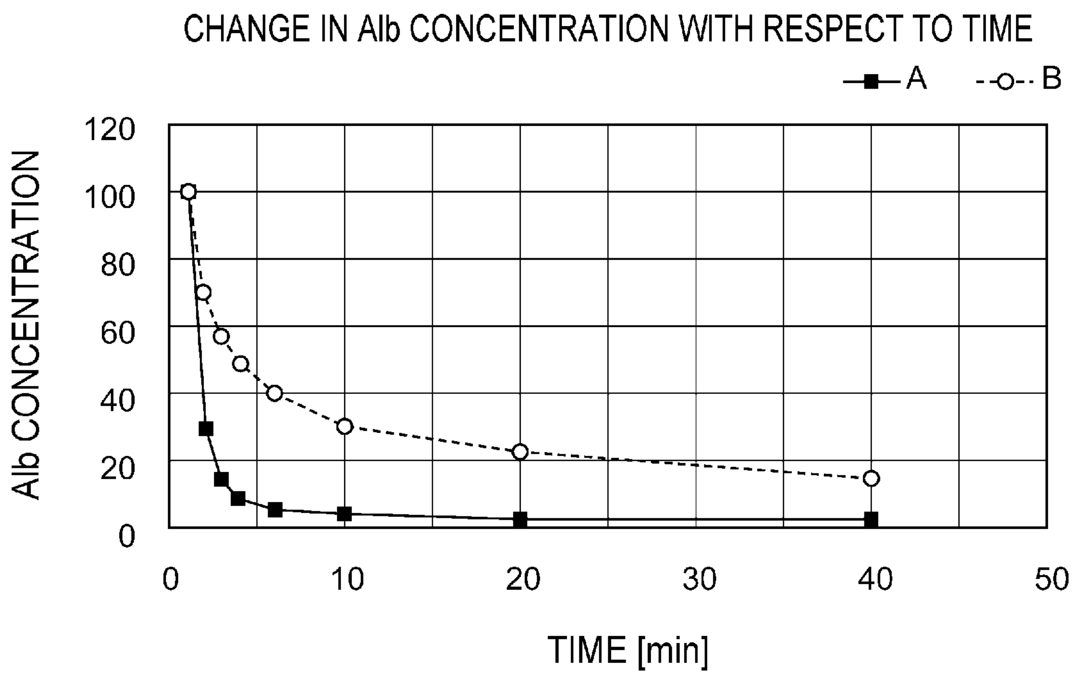
CHANGE IN Alb CONCENTRATION WITH RESPECT TO TIME
A
B
120
100
80
60
40
20
0
Alb CONCENTRATION
0
10
20
30
40
50
TIME [min]

Fig. 9

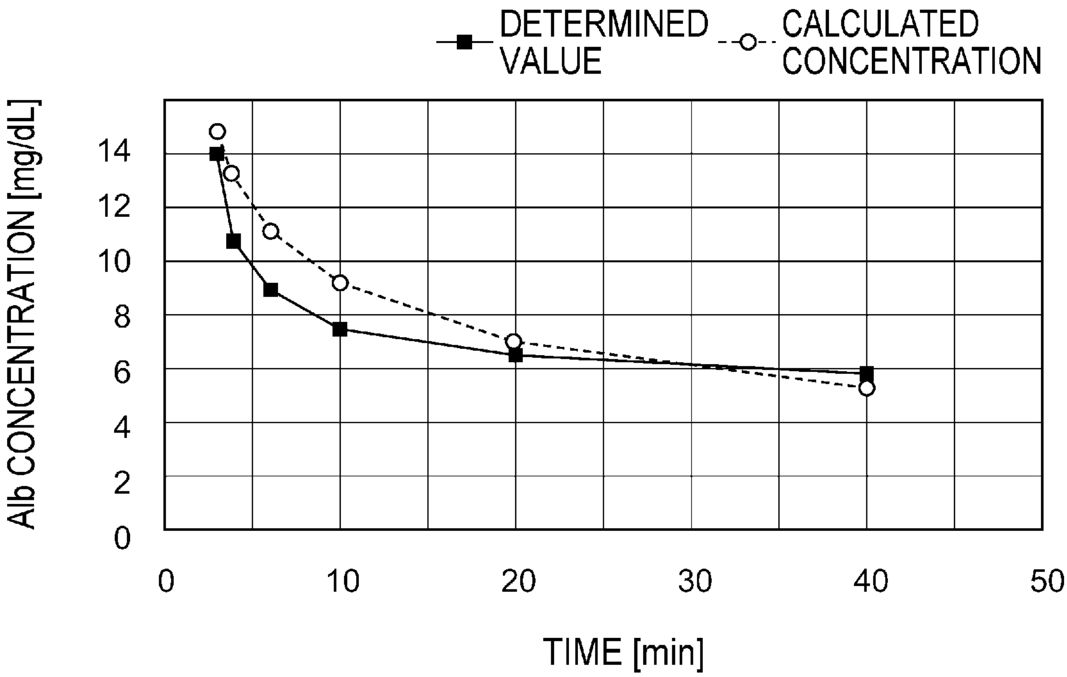
DETERMINED VALUE
CALCULATED CONCENTRATION
Alb CONCENTRATION [mg/dL]
14
12
10
8
6
4
2
0
0
10
20
30
40
50
TIME [min]

Fig. 10

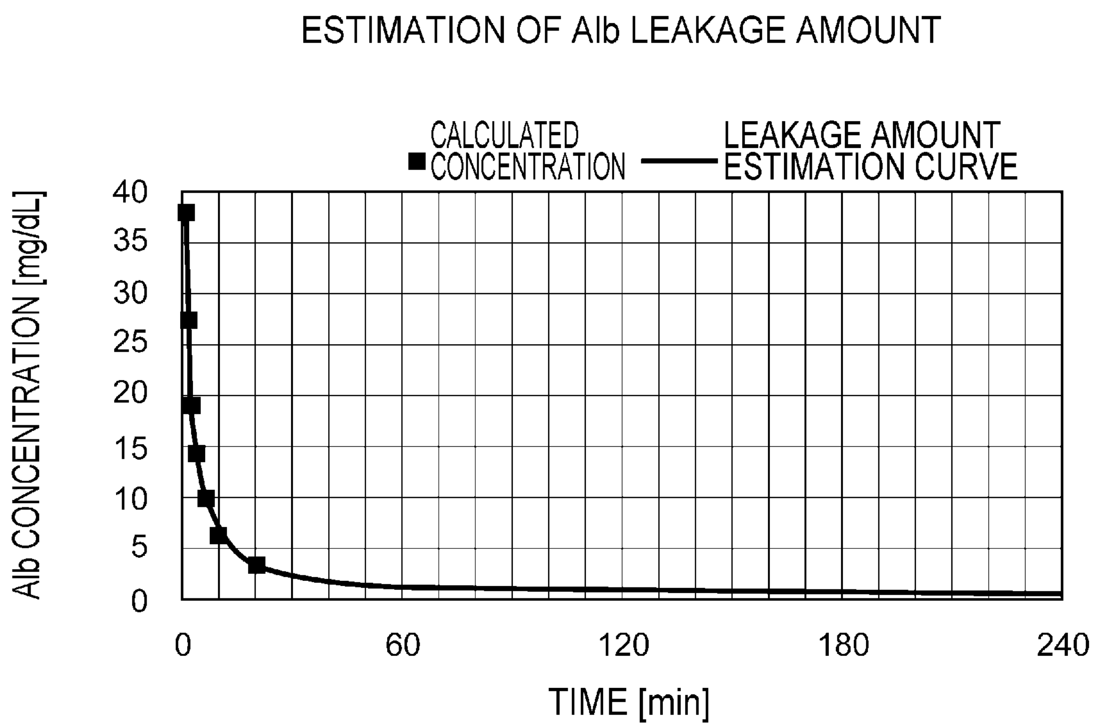
ESTIMATION OF Alb LEAKAGE AMOUNT
CALCULATED CONCENTRATION
LEAKAGE AMOUNT ESTIMATION CURVE
Alb CONCENTRATION [mg/dL]
40
35
30
25
20
15
10
5
0
0
60
120
180
240
TIME [min]

Fig. 11

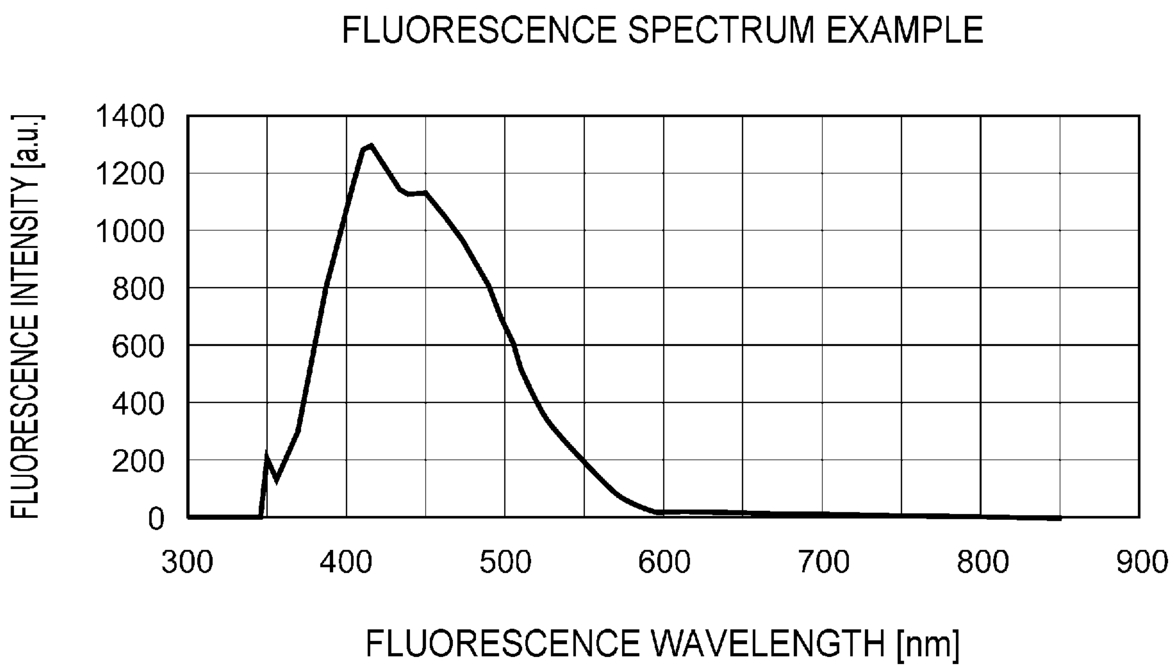
FLUORESCENCE SPECTRUM EXAMPLE
FLUORESCENCE INTENSITY [a.u.]
1400
1200
1000
800
600
400
200
0
300
400
500
600
700
800
900
FLUORESCENCE WAVELENGTH [nm]

Fig. 12

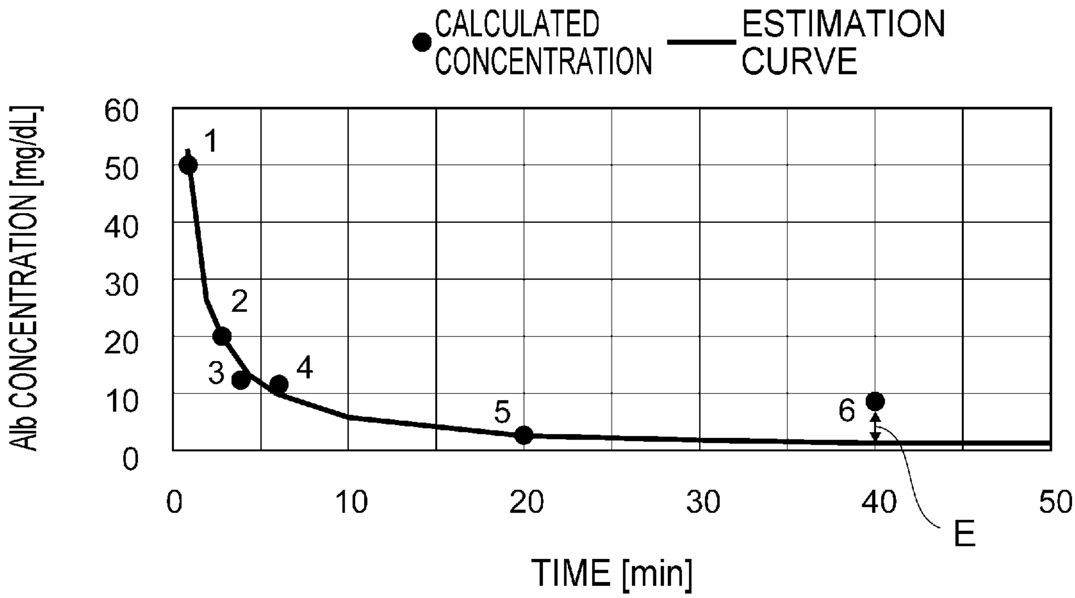
CALCULATED CONCENTRATION
ESTIMATION CURVE
Alb CONCENTRATION [mg/dL]
60
50
40
30
20
10
0
1
2
3
4
5
6
0
10
20
30
40
50
E
TIME [min]

Fig. 13

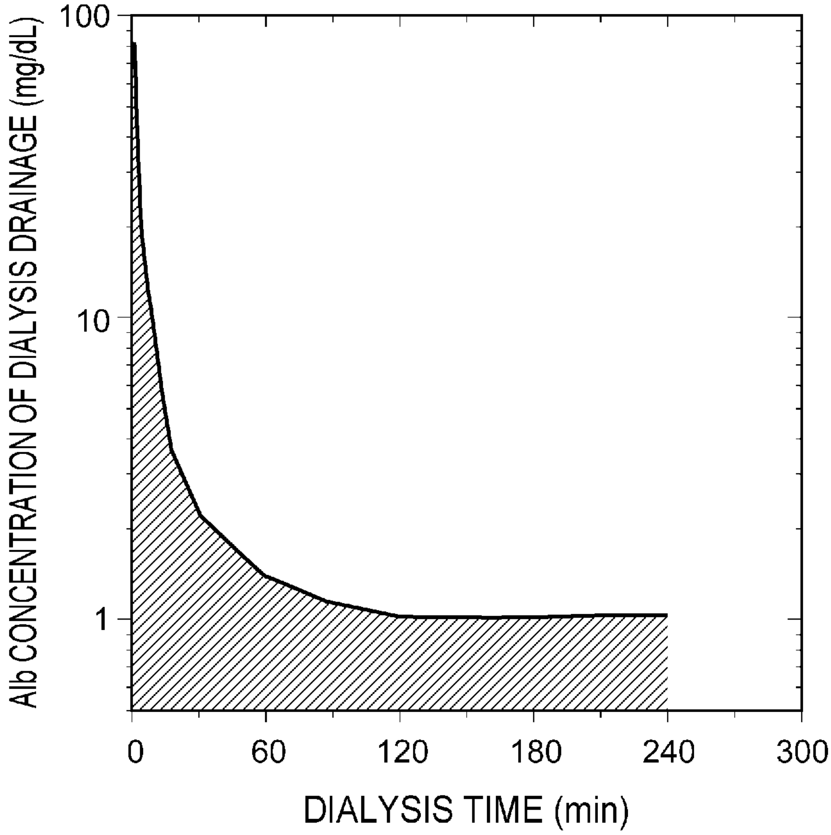
100
10
1
Alb CONCENTRATION OF DIALYSIS DRAINAGE (mg/dL)
0
60
120
180
240
300
DIALYSIS TIME (min)

Fig. 14

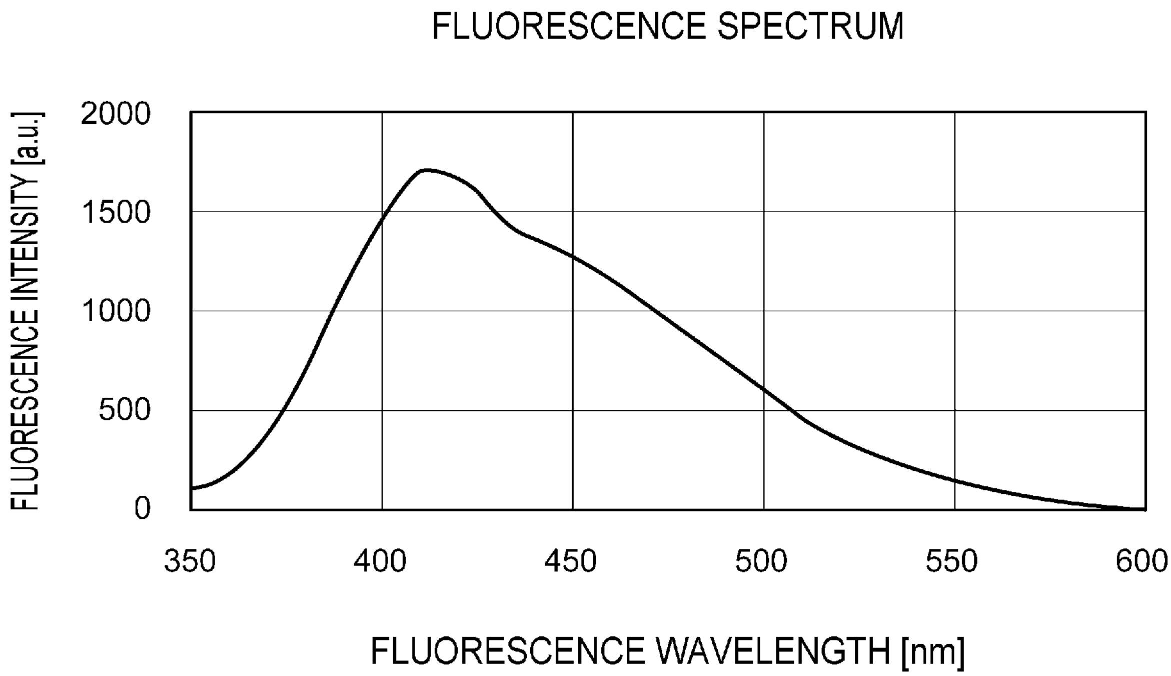
FLUORESCENCE SPECTRUM
FLUORESCENCE INTENSITY [a.u.]
2000
1500
1000
500
0
350
400
450
500
550
600
FLUORESCENCE WAVELENGTH [nm]

Fig. 15

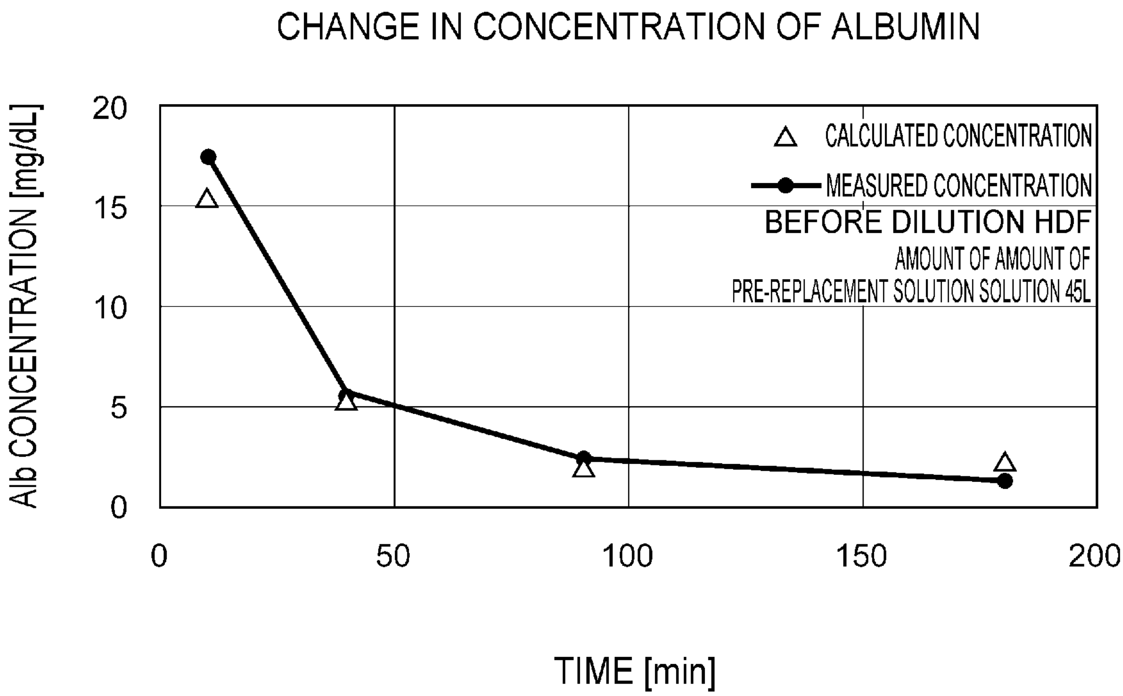
CHANGE IN CONCENTRATION OF ALBUMIN
Alb CONCENTRATION [mg/dL]
20
15
10
5
0
0
50
100
150
200
TIME [min]
CALCULATED CONCENTRATION
MEASURED CONCENTRATION
BEFORE DILUTION HDF
AMOUNT OF AMOUNT OF
PRE-REPLACEMENT SOLUTION SOLUTION 45L

Fig. 16

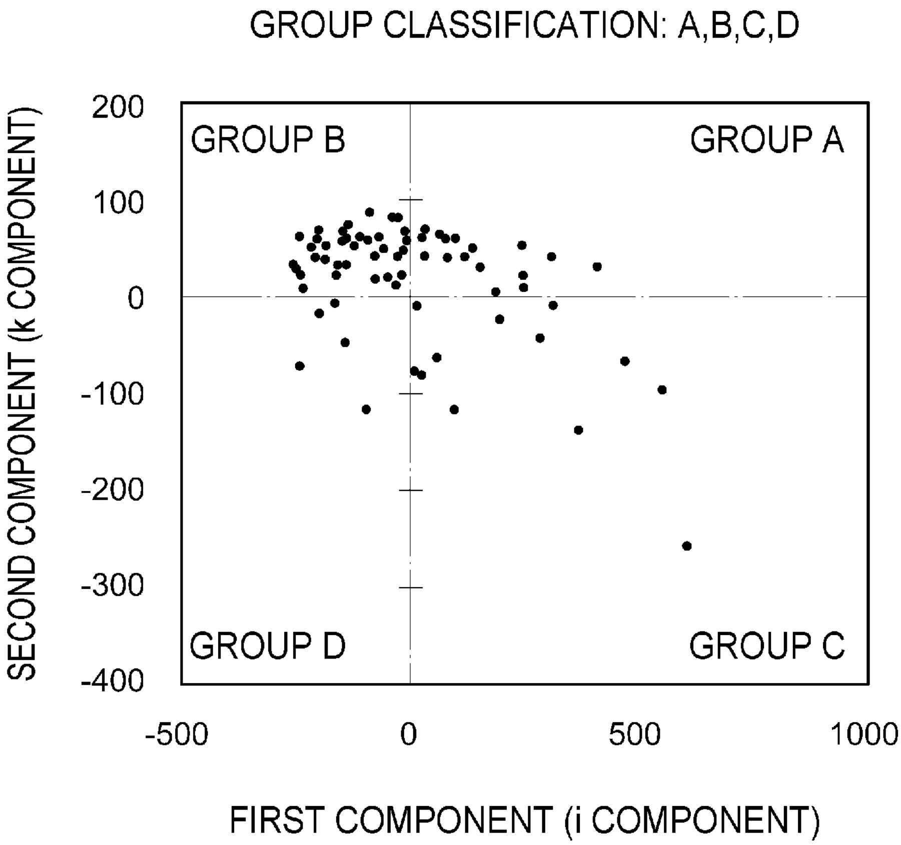
GROUP CLASSIFICATION: A,B,C,D
GROUP B
GROUP A
GROUP D
GROUP C
200
100
0
-100
-200
-300
-400
-500
0
500
1000
SECOND COMPONENT (k COMPONENT)
FIRST COMPONENT (i COMPONENT)

Fig. 17

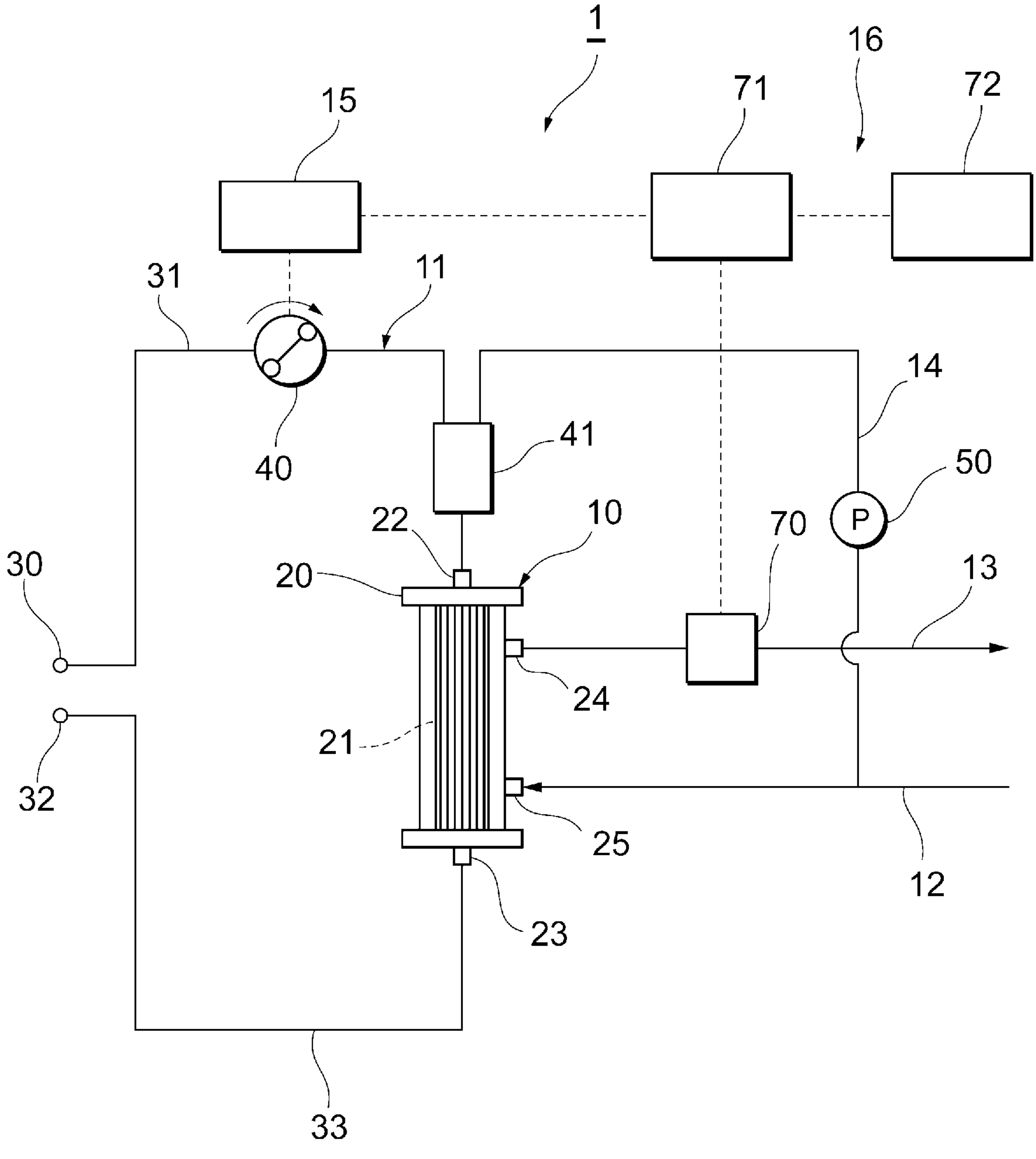

Fig. 18

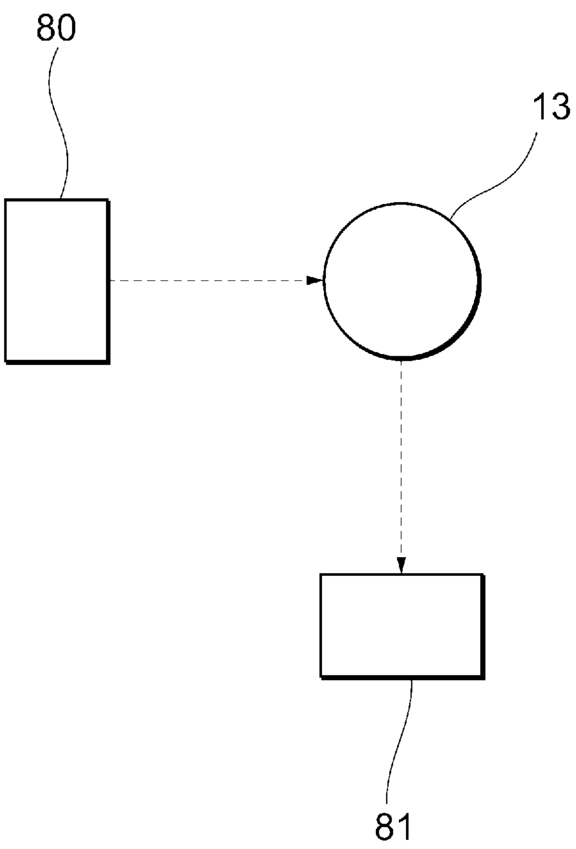

Fig. 19

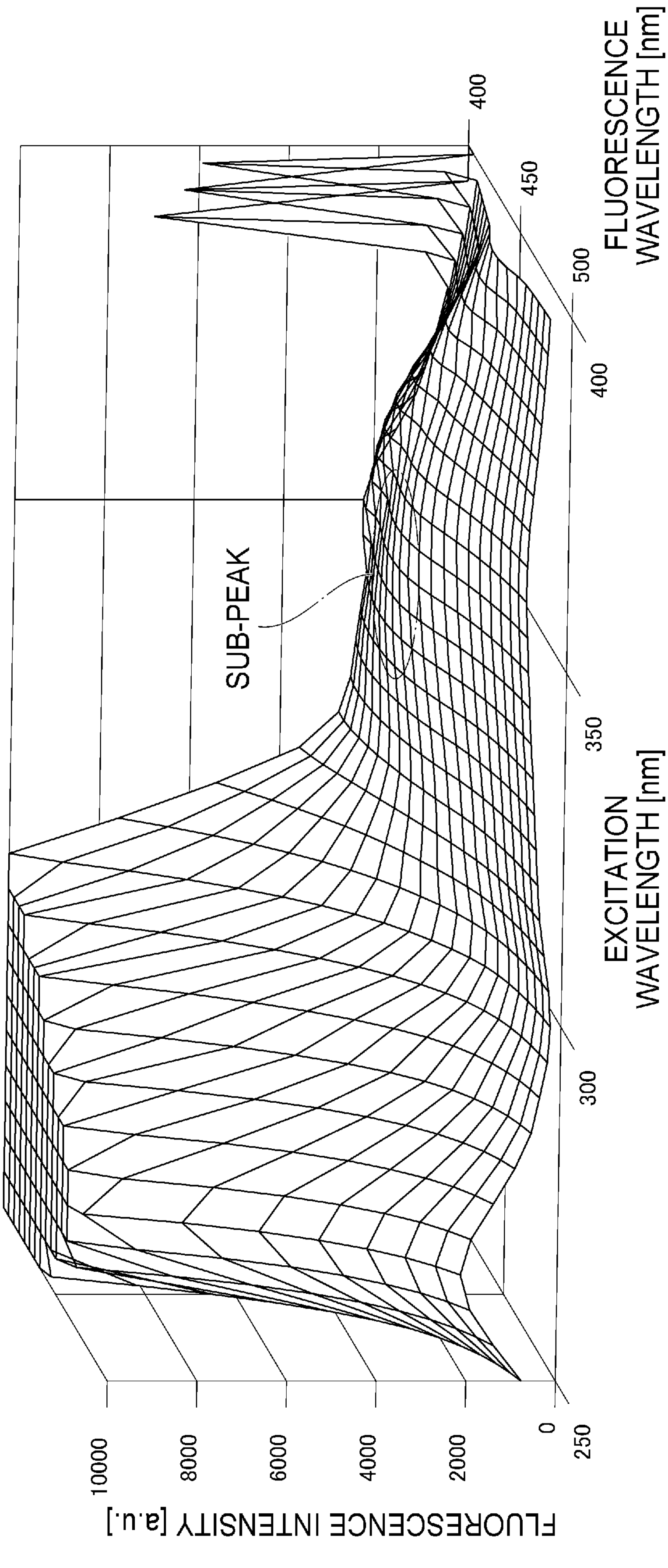
SUB-PEAK
FLUORESCENCE INTENSITY [a.u.]
10000
8000
6000
4000
2000
0
250
300
350
400
EXCITATION WAVELENGTH [nm]
400
450
500
FLUORESCENCE WAVELENGTH [nm]

Fig. 20

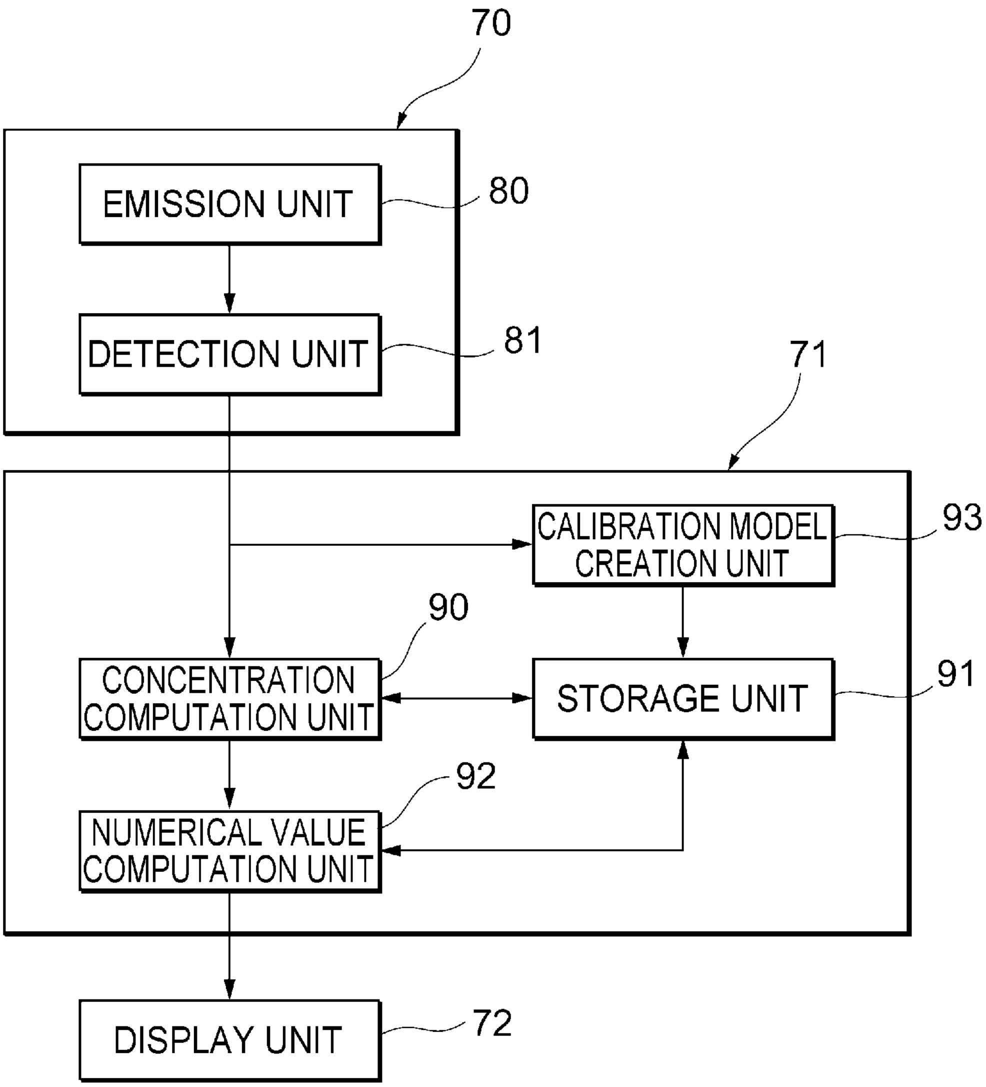
70
EMISSION UNIT
80
DETECTION UNIT
81
71
CALIBRATION MODEL CREATION UNIT
93
90
CONCENTRATION COMPUTATION UNIT
STORAGE UNIT
91
92
NUMERICAL VALUE COMPUTATION UNIT
DISPLAY UNIT
72

Fig. 21

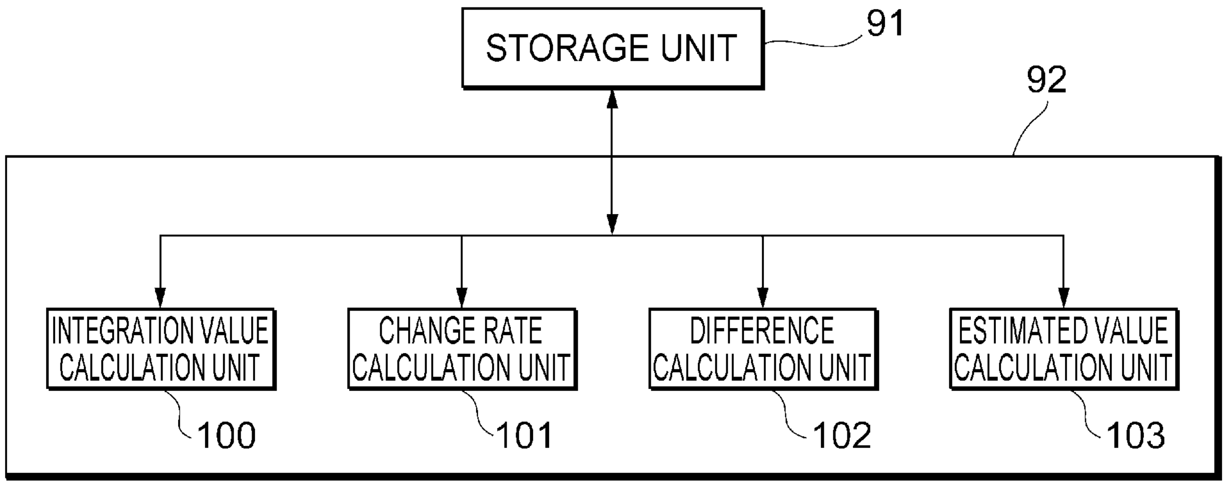
STORAGE UNIT
91
92
INTEGRATION VALUE CALCULATION UNIT
100
CHANGE RATE CALCULATION UNIT
101
DIFFERENCE CALCULATION UNIT
102
ESTIMATED VALUE CALCULATION UNIT
103

Fig. 22

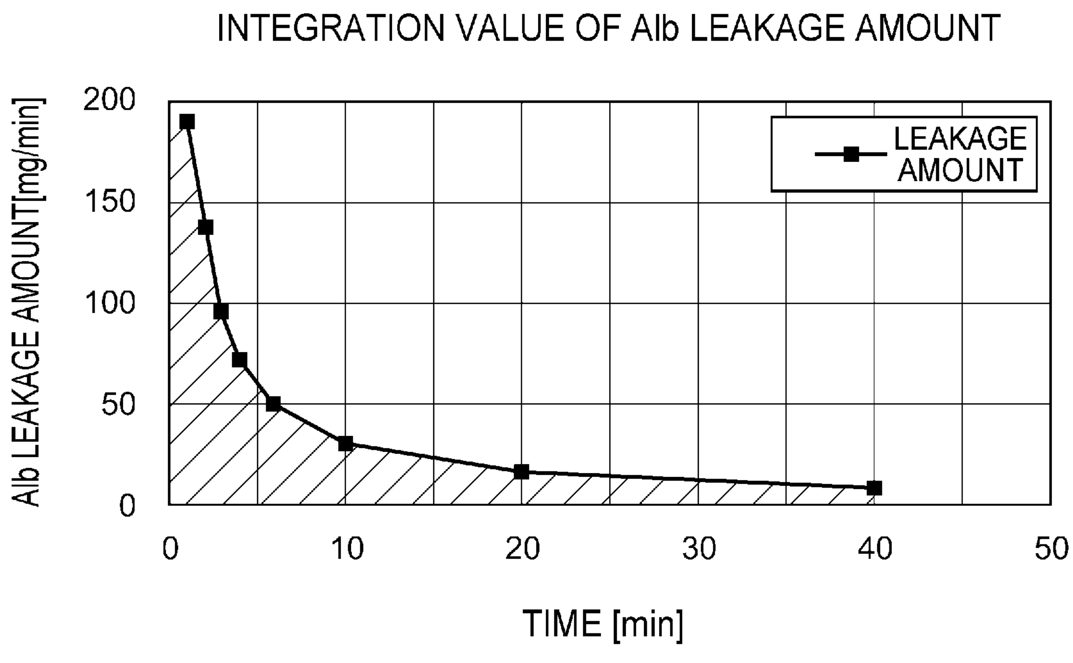
INTEGRATION VALUE OF Alb LEAKAGE AMOUNT
Alb LEAKAGE AMOUNT[mg/min]
200
150
100
50
0
0
10
20
30
40
50
TIME [min]
LEAKAGE AMOUNT

Fig. 23

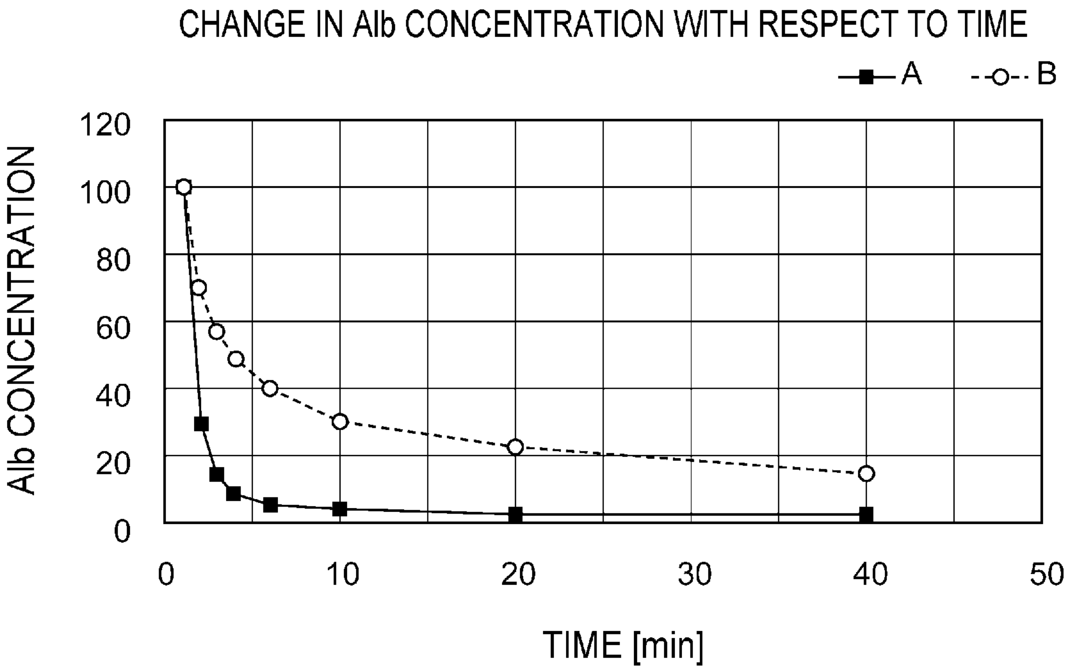
CHANGE IN Alb CONCENTRATION WITH RESPECT TO TIME
A
B
120
100
80
60
40
20
0
Alb CONCENTRATION
0
10
20
30
40
50
TIME [min]

Fig. 24

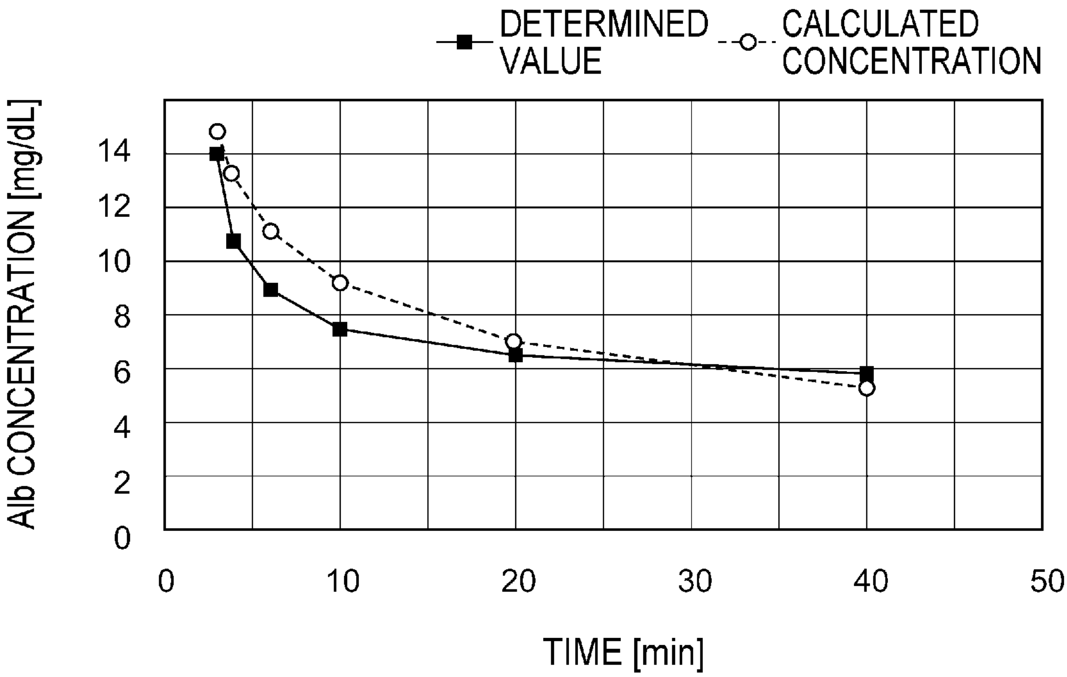

DETERMINED VALUE
CALCULATED CONCENTRATION
Alb CONCENTRATION [mg/dL]
14
12
10
8
6
4
2
0
0
10
20
30
40
50
TIME [min]

Fig. 25

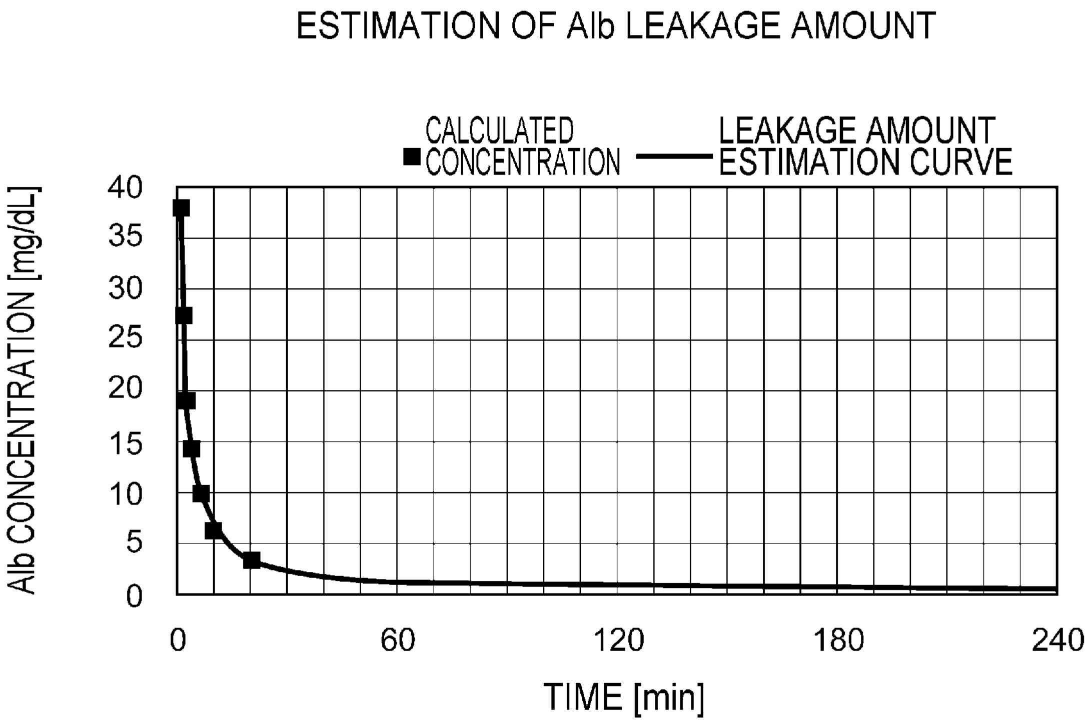
ESTIMATION OF Alb LEAKAGE AMOUNT
CALCULATED CONCENTRATION
LEAKAGE AMOUNT ESTIMATION CURVE
Alb CONCENTRATION [mg/dL]
40
35
30
25
20
15
10
5
0
0
60
120
180
240
TIME [min]

Fig. 26

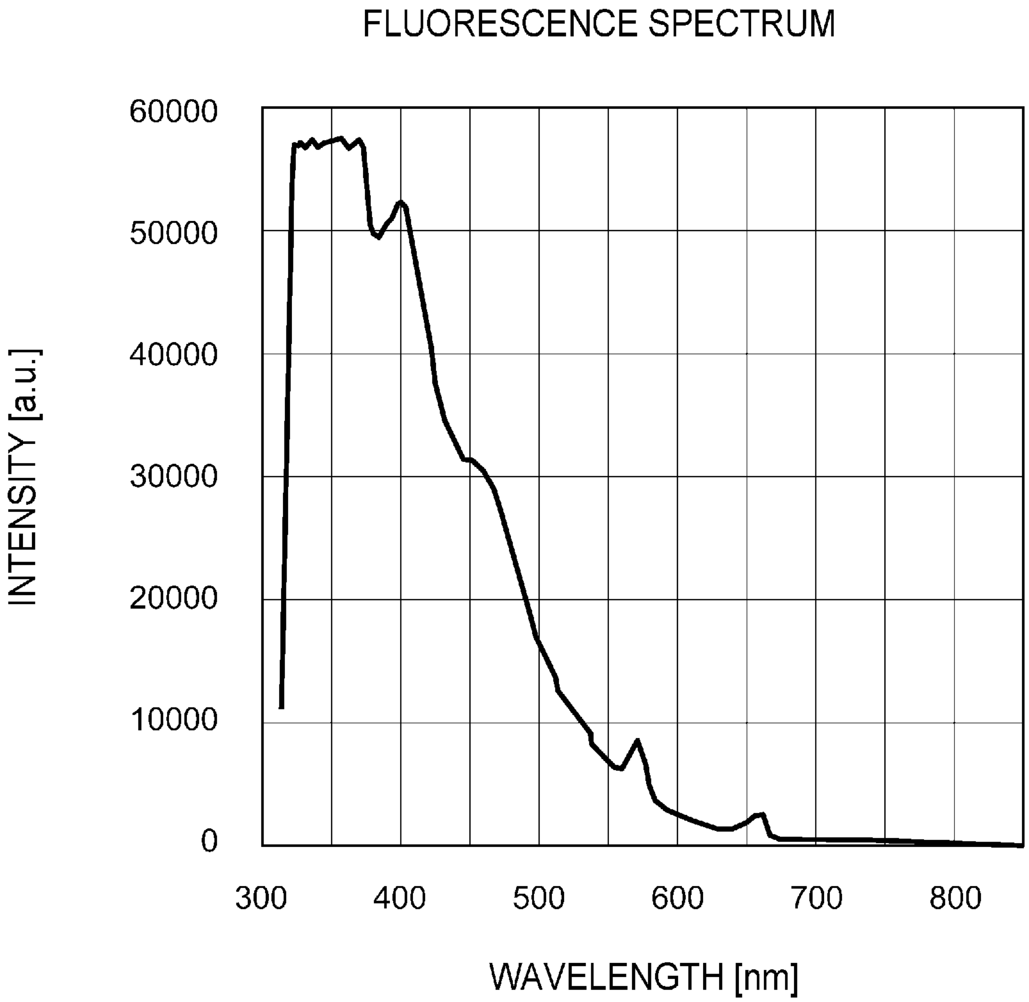
FLUORESCENCE SPECTRUM
60000
50000
40000
30000
20000
10000
0
INTENSITY [a.u.]
300
400
500
600
700
800
WAVELENGTH [nm]

Fig. 27

FLUORESCENCE WAVELENGTH RANGE, CORRELATION COEFFICIENT R AND ROOT MEAN SQUARE ERROR RMSE

| FLUORESCENCE WAVELENGTH [nm] | 310 ～ 850 | 320 ～ 850 | 330 ～ 850 | 340 ～ 850 | 350 ～ 850 | 340 ～ 750 | 340 ～ 780 | 340 ～ 800 | 340 ～ 830 | 340 ～ 850 |
|---|---|---|---|---|---|---|---|---|---|---|
| R | 0.9986 | 0.9988 | 0.9990 | 0.9990 | 0.9990 | 0.9998 | 0.9995 | 0.9991 | 0.9990 | 0.9998 |
| RMSE[mg/dL] | 1.07 | 1.10 | 0.884 | 0.866 | 0.940 | 0.650 | 0.677 | 0.843 | 0.874 | 0.970 |

Fig. 28

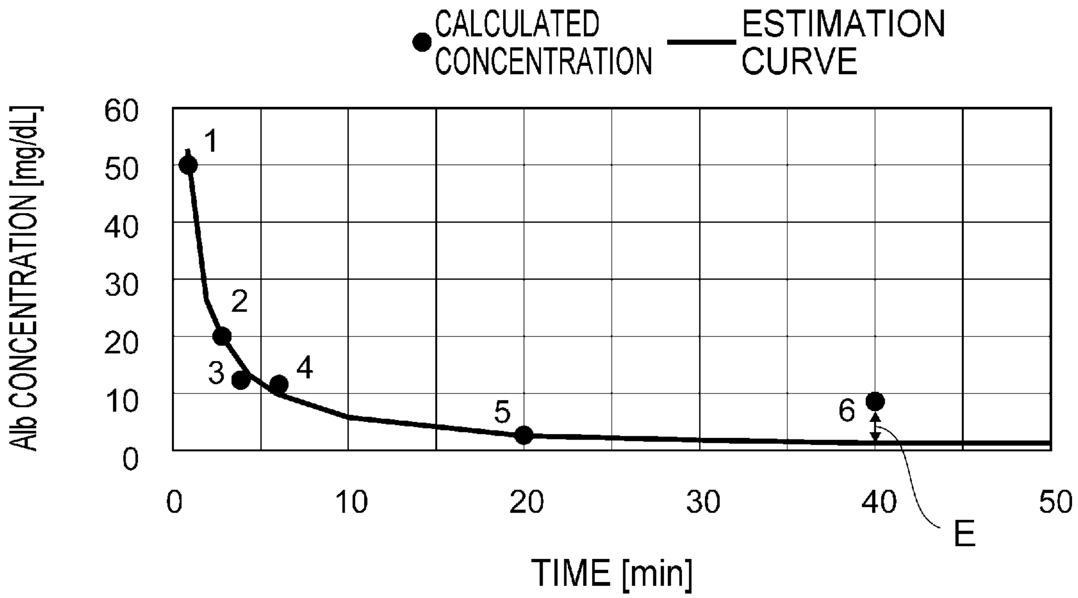
CALCULATED CONCENTRATION
ESTIMATION CURVE
Alb CONCENTRATION [mg/dL]
60
50
40
30
20
10
0
1
2
3
4
5
6
0
10
20
30
40
50
E
TIME [min]

Fig. 29

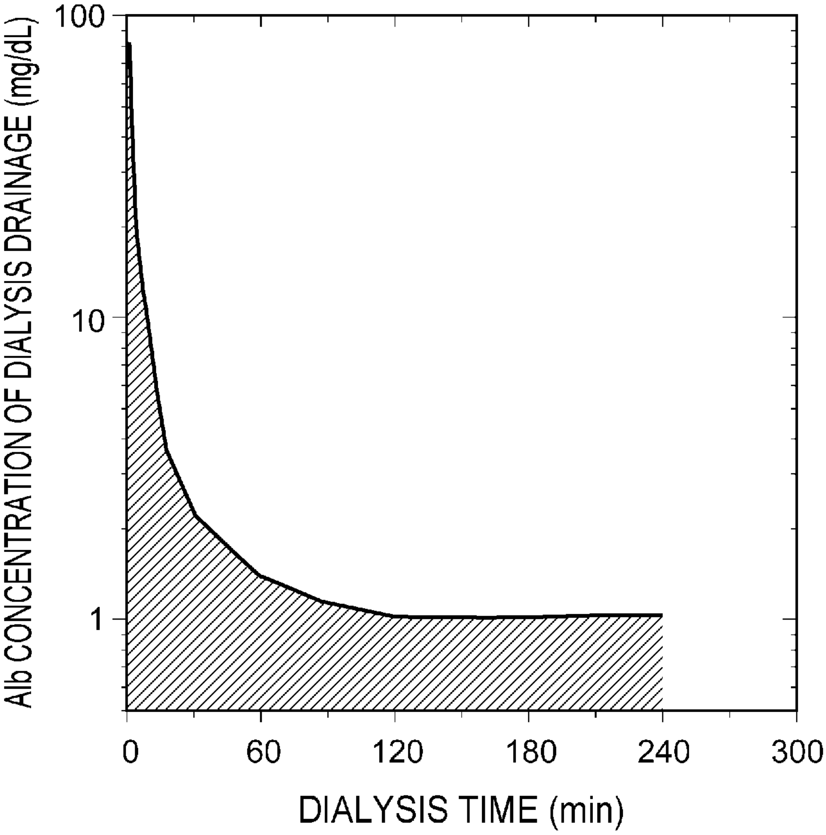
100
10
1
Alb CONCENTRATION OF DIALYSIS DRAINAGE (mg/dL)
0
60
120
180
240
300
DIALYSIS TIME (min)

Fig. 30

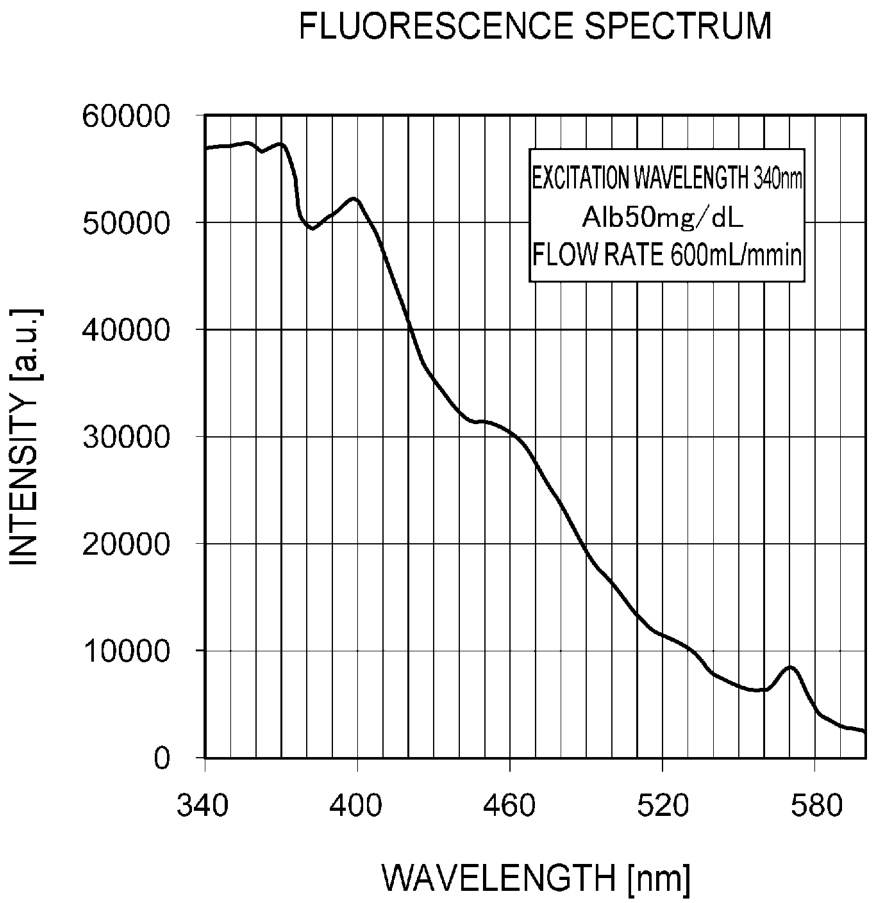
FLUORESCENCE SPECTRUM
EXCITATION WAVELENGTH 340nm
Alb50mg/dL
FLOW RATE 600mL/mmin
INTENSITY [a.u.]
60000
50000
40000
30000
20000
10000
0
340
400
460
520
580
WAVELENGTH [nm]

Fig. 31

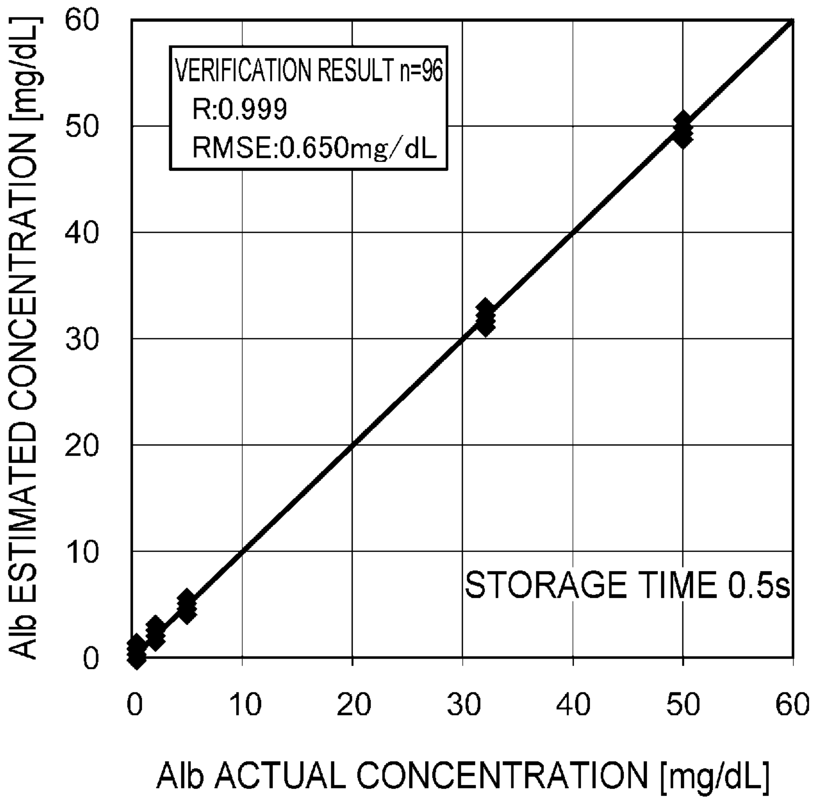
VERIFICATION RESULT n=96
R:0.999
RMSE:0.650mg/dL
STORAGE TIME 0.5s
Alb ESTIMATED CONCENTRATION [mg/dL]
Alb ACTUAL CONCENTRATION [mg/dL]
0
10
20
30
40
50
60

Fig. 32

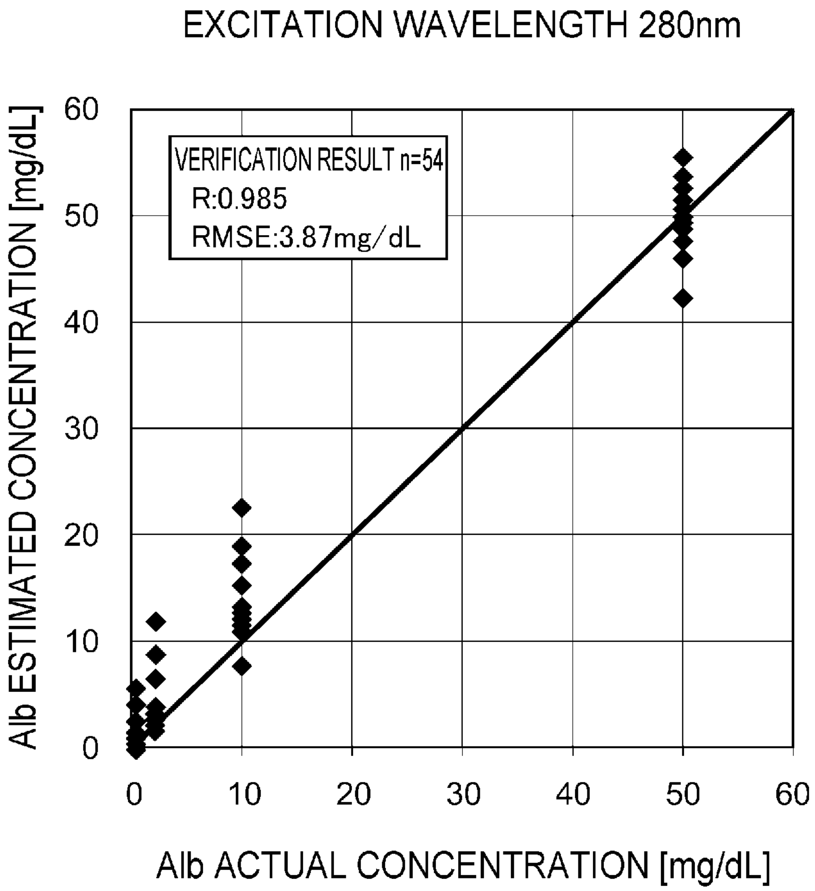
EXCITATION WAVELENGTH 280nm
VERIFICATION RESULT n=54
R:0.985
RMSE:3.87mg/dL
Alb ESTIMATED CONCENTRATION [mg/dL]
0
10
20
30
40
50
60
Alb ACTUAL CONCENTRATION [mg/dL]

Fig. 33

EXCITATION WAVELENGTH AND ROOT MEAN SQUARE ERROR (RMSE)

| | COMPARATIVE EXAMPLE 1 | EXAMPLE 2 | EXAMPLE 3 | EXAMPLE 4 | EXAMPLE 5 | EXAMPLE 6 | EXAMPLE 7 | COMPARATIVE EXAMPLE 2 |
|---|---|---|---|---|---|---|---|---|
| EXCITATION WAVELENGTH [nm] | 280 | 310 | 320 | 330 | 340 | 350 | 380 | 410 |
| RMSE[mg/dL] | 3.87 | 0.873 | 0.785 | 0.718 | 0.650 | 0.698 | 0.830 | 1.06 |

Fig. 34

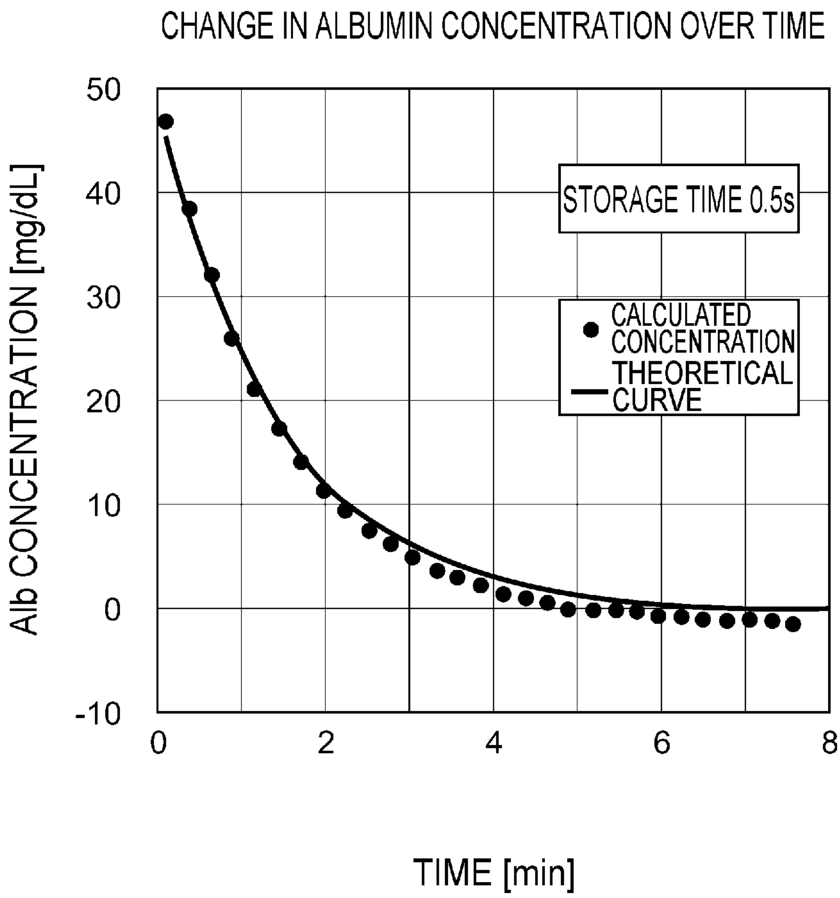
CHANGE IN ALBUMIN CONCENTRATION OVER TIME
Alb CONCENTRATION [mg/dL]
50
40
30
20
10
0
-10
0
2
4
6
8
TIME [min]
STORAGE TIME 0.5s
CALCULATED CONCENTRATION
THEORETICAL CURVE

**INTERNATIONAL SEARCH REPORT**

International application No.
PCT/JP2020/025023

A. CLASSIFICATION OF SUBJECT MATTER
G01N 21/64(2006.01)i; A61M 1/16(2006.01)i
FI: G01N21/64 Z; A61M1/16 111

According to International Patent Classification (IPC) or to both national classification and IPC

B. FIELDS SEARCHED

Minimum documentation searched (classification system followed by classification symbols)
G01N21/64; A61M1/16

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII)

C. DOCUMENTS CONSIDERED TO BE RELEVANT

| Category* | Citation of document, with indication, where appropriate, of the relevant passages | Relevant to claim No. |
|---|---|---|
| A | 藤田かおり 他，励起蛍光マトリクス計測に基づくデオキシニバレノール検知法の開発，食品総合研究所研究報告，2008, no. 72, PP. 23-30, entire text, all drawings, (FUJITA, Kaori et al., "Detection of deoxynivalenol in water solution by using three-dimensional excitation-emission matrix", Report of National Food Research Institute) | 1-36 |
| A | JP 5856741 B2 (NATIONAL AGRICULTURE AND FOOD RESEARCH ORGANIZATION) 10.02.2016 (2016-02-10) | 1-36 |
| A | JP 2014-518517 A (FRESENIUS MEDICAL CARE DEUTSCHLAND GMBH) 31.07.2014 (2014-07-31) | 1-36 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 11 September 2020 (11.09.2020) | 29 September 2020 (29.09.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2020/025023

| Patent Documents referred in the Report | Publication Date | Patent Family | Publication Date |
|---|---|---|---|
| JP 5856741 B2 | 10 Feb. 2016 | (Family: none) | |
| JP 2014-518517 A | 31 Jul. 2014 | US 2014/0098359 A1<br>WO 2012/140022 A1<br>EP 2510958 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2019118622 A
- JP 2019118624 A
- EP 2579910 A
- EP 2579911 A
- JP 2014518517 W
- JP 2015521492 W
- JP 2015146837 A